(19) Europäisches Patentamt
European Patent Office
Office européen des brevets

(11) EP 2 946 326 B1

(12) **EUROPEAN PATENT SPECIFICATION**

(45) Date of publication and mention
of the grant of the patent:
**18.05.2022 Bulletin 2022/20**

(21) Application number: **14704216.2**

(22) Date of filing: **21.01.2014**

(51) International Patent Classification (IPC):
**G16B 25/10** (2019.01)   **G16B 40/20** (2019.01)

(52) Cooperative Patent Classification (CPC):
**G16B 25/10**; G16B 40/20

(86) International application number:
**PCT/US2014/012390**

(87) International publication number:
**WO 2014/113815 (24.07.2014 Gazette 2014/30)**

(54) **SYSTEMS AND METHODS FOR GENE EXPRESSION ANALYSIS FOR PREDICTING PATIENT RESPONSE TO TARGETED THERAPIES**

SYSTEME UND VERFAHREN ZUR GENEXPRESSIONSANALYSE ZUR VORHERSAGE VON PATIENTENANTWORT AUF GEZIELTE THERAPIEN

SYSTÈMES ET MÉTHODES D'ANALYSE DE L'EXPRESSION GÉNÉTIQUE POUR LA PRÉDICTION DE REPONSE DE PATIENTS AUX THÉRAPIES CIBLÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2013 US 201361754892 P**
**12.03.2013 US 201361777716 P**

(43) Date of publication of application:
**25.11.2015 Bulletin 2015/48**

(73) Proprietor: **Life Technologies Corporation**
**Carlsbad, CA 92008 (US)**

(72) Inventor: **GEORGE, Wallace**
**Carlsbad, CA 92008 (US)**

(74) Representative: **Ziermann, Oliver et al**
**Life Technologies GmbH**
**Frankfurter Straße 129b**
**64293 Darmstadt (DE)**

(56) References cited:
**US-A1- 2003 207 278     US-A1- 2004 193 019**
**US-A1- 2011 307 427**

• **HUI ZOU ET AL: "Sparse Principal Component Analysis", JOURNAL OF COMPUTATIONAL AND GRAPHICAL STATISTICS, vol. 15, no. 2, 26 April 2004 (2004-04-26) , pages 265-286, XP55121469, ISSN: 1061-8600, DOI: 10.1198/106186006X113430**

**Description**

**FIELD**

The present disclosure generally relates to nucleic acid sequence data and in particular to systems, methods, software and computer-usable media for classifying metagenes (gene signatures) detected in the nucleic acid sequence data, to identify and select subsets of patients with likely disease or treatment response characteristics and to predict their expected treatment response.

**INTRODUCTION**

One of the overarching requirements for information-based Molecular Medical diagnostics and targeted personalized healthcare is the ability to identify biomarkers that makes it easy to classify and select responders to particular medical therapies and/or subsets of patients with increased probabilities for getting particular diseases. Thus the grand vision is twofold, namely:

> 1. To create classifiers to identify subsets of patients with increased probabilities for succumbing to particular health or medical conditions or getting particular diseases such as cancer or heart disease (Patient Disease Class Discovery).
> 2. To create Gene Expression (Gx) Classifiers that use a patient's Biomarkers, including their Gene Expression (Gx) information to help doctors select treatments personalized to them, eliminating pointless and often harsh treatments, and reducing adverse drug reactions to medical care informed by the patient's specific genetic makeup (Patient Treatment Class Prediction).

In this context, the term Biomarker is used as a generalization of its official NIH definition. That is, the term Biomarker is being used here to mean a characteristic (including demographic profile and lifestyle status) that can be objectively measured and/or evaluated as a clinical indicator of a patient's current or past health, normal biologic processes, pathogenic processes, and/or pharmacologic responses to a potential therapeutic intervention. It includes factors such as weight, age, blood test results, gender, and Gene Expression information. As a result, it's important to identify and select highly relevant sets of genes (Gene Signatures, Biomarkers or metagenes), which make it easy to perform Patient Disease Class Discovery and Patient Treatment Class Prediction (i.e. to discriminate between responders and non-responders to particular medical treatments). Therefore, finding a good set of genes for this purpose is a critical requirement for achieving the dream of effective Personalized Medicine. Thus, developing algorithms that can reliably identify critical Gene Signatures (class discovery), and Gene Expression Classifiers that can be used to predict or identify responders (Patient Treatment Class Prediction), are key elements in this convergence of genomics and informatics.

However, despite intensive efforts this remains a difficult task. Even small improvements in the performance and flexibility of genomic classifier algorithms could herald a new era of biomedical research. The fundamental problem is that Gene Expression (Gx) information can be associated with thousands of genes and available patient clinical response information typically numbers hundreds of patients or less. This is a very under-determined problem if we think of the critical genes as the "unknowns" and the data for each patient as a new "equation." Thus, the set of critical biomarkers/genes forming a Gene Signature is not unique. As a result, the selection of a particular Gene Signature from a given patient data set can be easily biased by noise and idiosyncrasies in the particular patient dataset. This is a problem that the Patient Disease Class Discovery (Gene Signature Identification) algorithm must address.

The problem of Gene Signature Identification and Gx Patient Response Classification or Prediction has attracted a wide variety of machine learning approaches. These approaches can be broadly classed as unsupervised or supervised (intermediate classes are called semi-supervised). Unsupervised machine learning algorithms try to find the unrevealed structure in an unlabeled training dataset. Supervised machine learning algorithms use labeled training data to categorize the structure of a training dataset. Supervised learning algorithms typically categorize the structure of a training dataset by approaching the problem as either a "classification problem" or a "prediction problem". The classification approach tries to assign the new patient into one of the labeled patient classes, used in the training set that the patient seems to most closely belong to. Because of this discrete class output the classification approach produces a classifier. The prediction approach leads to a predicted continuous regression function as an intermediate output. Since this continuous function can be thresholded to produce discrete class outputs, both approaches will be thought of as producing classifiers. The classification approach tries to identify the true class of an unlabeled patient. The prediction approach tries to predict the true class of an unlabeled patient by calculating the value(s) of characteristic(s) that are used to later identify the patient's true class.

The fundamental characteristic of the patient classification problem is that the number of possible patient features (such as genomic profile, gene expression profile, patient demographics, medical/health history, and/or personal and lifestyle characteristics) are typically much larger than the number of patients in most training sets. Many of these features can be highly correlated with each other. As a result, the set of critical features (e.g. gene signatures, gene expression, patient demographics, medical/health history, and/or personal and lifestyle characteristics) identified by the various algorithms to classify a particular patient condition can differ greatly from each other. This is true, both in the number and in the identity of the features chosen. Computationally, since the problem is very "under-determined" there can be many sets of features that can result in nearly equivalent classification performance.

As an example, we will look at the case of breast cancer. There are three genomic assays currently in use for breast cancer: MammaPrint, Oncotype DX, and Mammostrat Dx. While all three tests are somewhat similar, there are differences:

The Oncotype Dx test is used to estimate a woman's risk of recurrence of early-stage, hormone-receptor-positive (HER2-positive) breast cancer, and how likely she is to benefit from chemotherapy after breast cancer surgery. It analyzes the activity of 21 genes. The MammaPrint Dx test is used to estimate a women's recurrence risk for early-stage breast cancer (both HER2-positive and HER2-negative). It analyzes the activity of 70 genes. The Mammostrat DX test is used to estimate a woman's risk of recurrence of early-stage, HER2 - positive breast cancer. It analyzes the activity of only 5 genes.

Not only is the number of genes used for the breast cancer patient classification dramatically different, there is little over-lap between the genes chosen for each of these tests.

In general, there is a high level of cross-correlation among the patients' features. Thus, we can expect that a much smaller set of meta-features (weighted groupings of the original features) that are approximately independent or orthogonal to each other exists. This smaller number of meta-features represents the true underlying number of degrees of freedom (df) for the original feature set and training data. Therefore, one of the critical tasks for a successful classification algorithm can be to approximately identify this smaller set of meta-features, then use them to more reliably, and in an over-determined manner, perform patient classification.

Conventional techniques do not facilitate easy correlation of candidate Gene Signatures or metagenes with the wealth of information that is currently available that can provide the interpretive context to identify and select the highly relevant sets of Patient Treatment Class Prediction and Patient Disease Class Discovery genes, that is currently available. This is due to the enormous amount of information being generated by researchers and the lack of adequate tools to organize the information in a manner that facilitates their analysis.

As such, there is a need for systems and methods that can reliably identify critical Gene Signatures (Class Discovery), and Gene Expression Classifiers that can be used to predict or identify responders (Class Prediction).

## DRAWINGS

For a more complete understanding of the principles disclosed herein, and the advantages thereof, reference is now made to the following descriptions taken in conjunction with the accompanying drawings, in which:

Figure 1 is a block diagram that illustrates a computer system, in accordance with various embodiments.
Figure 2 is a schematic diagram of a system for reconstructing a nucleic acid sequence, in accordance with various embodiments.
Figure 3 is a schematic diagram of a system for predicting patient response to targeted therapies, in accordance with various embodiments.
Figure 4 is a schematic diagram detailing the operation of the gene signature identification engine, in accordance with various embodiments.
Figures 5 and 6 are exemplary flowcharts showing a method for predicting patient response to targeted therapies, in accordance with various embodiments.
Figure 7 is an exemplary example of a sparse eigenvector matrix, in accordance with various embodiments.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

## DESCRIPTION OF VARIOUS EMBODIMENTS

Embodiments of systems and methods for identifying critical Gene Signatures (Patient Disease Class Discovery) and predicting responders (Patient Treatment Class Prediction) to medical treatments using nucleic acid sequence data are described in this specification.

Research into fast and efficient nucleic acid (e.g., genome, exome, etc.) sequence assembly, analysis and interpretation methods is vital to the sequencing industry as Next Generation Sequencing (NGS) technologies can provide ultra-high throughput nucleic acid sequencing. As such sequencing systems incorporating NGS technologies can produce a large number of short sequence reads in a relatively short amount time. Sequence assembly methods must be able to assemble and/or map a large number of reads quickly and efficiently (i.e., minimize use of computational resources). For example, the sequencing of a human size genome can result in tens or hundreds of millions of reads that need to be assembled before they can be further analyzed to determine their biological, diagnostic and/or therapeutic relevance.

Exemplary applications of NGS technologies include, but are not limited to: genomic variant (e.g., indels, copy number variations, single nucleotide polymorphisms, etc.) detection, resequencing, gene expression analysis and genomic profiling.

A wealth of nucleic acid sequence information is now available in sequence databases, both public and private. For example, public databases of metabolic, genetic and physiological pathways of various organisms (e.g., Munich Information Center for Protein Sequences (MIPS), NCBI's Single Nucleotide Polymorphism database (dbSNP), etc.) and some genes (e.g., Kyoto Encyclopedia of Genes and Genomes (KEGG), etc.) have been developed largely from the published literature of many traditional low-throughput experimental studies. An advantage of this abundance of data is that improved diagnostic testing and genomics guided therapeutic regimens (e.g., drugs, surgery, radiation therapy, medical devices, diet, psychiatric therapy, etc.) will be possible as new information about how an individuals' genetic and epigenetic profile correlates to risk factors for disease, drug targets, protein therapeutics, devices, treatment protocols, and the like are identified and characterized. In addition, because relatively small differences in the genetic makeup (genotype), gene expression, or epigenetic status of individuals can result in large differences in physical characteristics (phenotype), some diagnostic testing and therapeutic regimens may work better with some individuals than with others, and in some cases deleterious effects can be avoided. With knowledge of how different genotypes or other genetic and epigenetic factors affect the function of a individual's various biological pathways (e.g., metabolic, signaling, regulation, etc.), diagnostic tests and treatment regimens can potentially be customized based on genetic and epigenetic information associated with the specific individual being treated.

While the quantity of nucleic acid sequence data that one can gather using conventional sequencing techniques is very large, it can often not be presented or analyzed in the most useful context. The diagnostic and therapeutic relevance of genetic and epigenetic data can often be best determined by its relationship to other pieces of information. For example, knowing that a particular genetic mutation (e.g., SNP, Indel, CNV, etc.) affects a particular metabolic or physiological pathway that plays a role in or otherwise affects the inception, progression, or treatment of a particular disease can be clinically important information. In addition, there is a need to correlate this data with various types of clinical data, for example, a patient's age, sex, weight, stage of clinical development, stage of disease progression, etc. In the various embodiments, a patient may be a human or animal, or any entity with a genomic structure.

The section headings used herein are for organizational purposes only and are not to be construed as limiting the described subject matter in any way.

It will be appreciated that there is an implied "about" prior to the temperatures, concentrations, times, number of bases, coverage, etc. discussed in the present teachings, such that slight and insubstantial deviations are within the scope of the present teachings. In this application, the use of the singular includes the plural unless specifically stated otherwise. Also, the use of "comprise", "comprises", "comprising", "contain", "contains", "containing", "include", "includes", and "including" are not intended to be limiting. It is to be understood that both the foregoing general description and the following detailed description are exemplary and explanatory only and are not restrictive of the present teachings.

Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. The techniques and procedures described herein are generally performed according to conventional methods well known in the art and as described in various general and more specific references that are cited and discussed throughout the instant specification. *See, e.g.,* Sambrook et al., Molecular Cloning: A Laboratory Manual (Third ed., Cold Spring Harbor Laboratory Press, Cold Spring Harbor, N.Y. 2000). The nomenclatures utilized in connection with, and the laboratory procedures and techniques described herein are those well known and commonly used in the art.

As used herein, "a" or "an" means "at least one" or "one or more."

A "system" denotes a set of components, real or abstract, comprising a whole where each component interacts with or is related to at least one other component within the whole.

A "biomolecule" is any molecule that is produced by a biological organism, including large polymeric molecules such as proteins, polysaccharides, lipids, and nucleic acids (DNA and RNA) as well as small molecules such as primary metabolites, secondary metabolites, and other natural products.

The phrase "next generation sequencing" or NGS refers to sequencing technologies having increased throughput as compared to traditional Sanger- and capillary electrophoresis-based approaches, for example with the ability to generate hundreds of thousands of relatively small sequence reads at a time. Some examples of next generation sequencing techniques include, but are not limited to, sequencing by synthesis, sequencing by ligation, and sequencing by hybridization. More specifically, the Ion Torrent Personal Genome Machine (PGM) and SOLiD Sequencing System of Life Technologies Corp. provide massively parallel sequencing with enhanced accuracy. The SOLiD System and associated workflows, protocols, chemistries, etc. are described in more detail in PCT Publication No. WO 2006/084132, entitled "Reagents, Methods, and Libraries for Bead-Based Sequencing," international filing date February 1, 2006, U.S. Patent Application Serial No. 12/873,190, entitled "Low-Volume Sequencing System and Method of Use," filed on August 31, 2010, and U.S. Patent Application Serial No. 12/873,132, entitled "Fast-Indexing Filter Wheel and Method of Use," filed on August 31, 2010.

The phrase "sequencing run" refers to any step or portion of a sequencing experiment performed to determine some information relating to at least one biomolecule (e.g., nucleic acid molecule).

It is well known that DNA (deoxyribonucleic acid) is a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. It is also known that certain pairs of nucleotides specifically bind to one another in a complementary fashion (called complementary base pairing). That is, adenine (A) pairs with thymine (T) (in the case of RNA, however, adenine (A) pairs with uracil (U)), and cytosine (C) pairs with guanine (G). In various embodiments, the nucleotides can be in a modified form, such as methylated C.

When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "nucleic acid sequence," "genomic sequence," "genetic sequence," "fragment read," "fragment sequence," "sequence read," or "nucleic acid sequencing read" denotes any information or data that is indicative of the order of the nucleotide bases (e.g., adenine, guanine, cytosine, and thymine/uracil) in a molecule (e.g., whole genome, whole transcriptome, exome, oligonucleotide, polynucleotide, fragment, etc.) of DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

The phrase "ligation cycle" refers to a step in a sequence-by-ligation process where a probe sequence is ligated to a primer or another probe sequence.

The phrase "color call" refers to an observed dye color resulting from the detection of a probe sequence after a ligation cycle of a sequencing run.

The phrase "color space" refers to a nucleic acid sequence data schema where nucleic acid sequence information is represented by a set of colors (e.g., color calls, color signals, etc.) each carrying details about the identity and/or positional sequence of bases that comprise the nucleic acid sequence. For example, the nucleic acid sequence "ATCGA" can be represented in color space by various combinations of colors that are measured as the nucleic acid sequence is interrogated using optical detection-based (e.g., dye-based, etc.) sequencing techniques such as those employed by the SOLiD System. That is, in various embodiments, the SOLiD System can employ a schema that represents a nucleic acid fragment sequence as an initial base followed by a sequence of overlapping dimers (adjacent pairs of bases). The system can encode each dimer with one of four colors using a coding scheme that results in a sequence of color calls that represent a nucleotide sequence.

The phrase "base space" refers to a nucleic acid sequence data schema where nucleic acid sequence information is represented by the actual nucleotide base composition of the nucleic acid sequence. For example, the nucleic acid sequence "ATCGA" is represented in base space by the actual nucleotide base identities (e.g., A, T/or U, C, G) of the nucleic acid sequence.

The phrase "flow space" refers to a nucleic acid sequence data schema wherein nucleic acid sequence information is represented by nucleotide base identifications (or identifications of known nucleotide base flows) coupled with signal or numerical quantification components representative of nucleotide incorporation events for the nucleic acid sequence. The quantification components may be related to the relative number of continuous base repeats (e.g., homopolymers) whose incorporation is associated with a respective nucleotide base flow. For example, the nucleic acid sequence "ATTTGA" may be represented by the nucleotide base identifications A, T, G and A (based on the nucleotide base flow order) plus a quantification component for the various flows indicating base presence / absence as well as possible existence of homopolymers. Thus for "T" in the example sequence above, the quantification component may correspond to a signal or numerical identifier of greater magnitude than would be expected for a single "T" and may be resolved to indicate the presence of a homopolymer stretch of "T"s (in this case a 3-mer) in the "ATTTGA" nucleic acid sequence.

A "polynucleotide", "nucleic acid", or "oligonucleotide" refers to a linear polymer of nucleosides (including deoxyribonucleosides, ribonucleosides, or analogs thereof) joined by internucleosidic linkages. Typically, a polynucleotide comprises at least three nucleosides. Usually oligonucleotides range in size from a few monomeric units, e.g. 3-4, to several hundreds of monomeric units. Whenever a polynucleotide such as an oligonucleotide is represented by a sequence of letters, such as "ATGCCTG," it will be understood that the nucleotides are in 5'->3' order from left to right and that "A" denotes deoxyadenosine, "C" denotes deoxycytidine, "G" denotes deoxyguanosine, and "T" denotes thymidine, unless otherwise noted. The letters A, C, G, and T may be used to refer to the bases themselves, to nucleosides, or to nucleotides comprising the bases, as is standard in the art.

The phrase "patient features," denotes a variety of information specific to an individual patient including, but not limited to, his/her genomic profile (e.g., genomic signature, biomarker profile, etc.), gene expression profile, patient demographics, medical/health history, and/or personal and lifestyle characteristics.

The phrase "genomic signatures," "gene signatures" or "metagenes" denotes a single gene or a set of genes (e.g., biomarkers) their associated variants and biological expressions (e.g., gene expression profile) thereof that have biological or therapeutic relevance. They represent a set of genes (and expressions thereof) that have been identified to make it easier for classification systems or algorithms to perform Patient Disease Class Discovery (i.e., to identify subsets of patients with increased probabilities for succumbing to particular health conditions or getting particular diseases such as cancer or heart disease) and/or Patient Treatment Class Prediction (i.e., to discriminate between responders and non-responders to particular medical treatments). The term "biomarker" is being used in this context as a generalization of its official NIH definition. That is, as used herein, the term Biomarker means a characteristic that can be objectively measured and/or evaluated as a clinical indicator of a patient's current or past health, normal biologic processes, pathogenic processes, and/or pharmacologic responses to a potential therapeutic intervention.

Gene Expression (Gx) and biomarker data used to create Gene Signatures can be obtained from multivariate real-time/digital/qPCR array data (such as TaqMan Array Cards data or miRNA profiling data from OpenArray), array-based methods (e.g., microarrays, etc.), Capillary Electrophoresis Sequencing (such as Life Technologies' 3730/3500/3130 DNA Analyzer) and/or Next Generation Sequencing (NGS) -based Gene Expression Profiling data (such as from Life Technologies' Ion Torrent System or SOLID™ Sequencing System).

The phrase "genomic variants" or "genome variants" denote a single or a grouping of genes (in DNA or RNA) that have undergone changes as referenced against a particular species or sub-populations within a particular species due to mutations, recombination/crossover or genetic drift. Examples of types of genomic variants include, but are not limited to: single nucleotide polymorphisms (SNPs), copy number variations (CNVs), insertions/deletions (Indels), inversions, etc.

Genomic variants can be identified using a variety of techniques, including, but not limited to: array-based methods (e.g., DNA microarrays, etc.) real-time/digital/quantitative PCR instrument methods and whole or targeted nucleic acid sequencing. With nucleic acid sequencing, coverage data can be available at single base resolution. Nucleic acid sequencing systems such as the Life Technologies/Ion Torrent Personal Genome Machine (PGM), SOLID™ Sequencing System and 3730/3500/3130 series DNA Analyzers can be used to sequence nucleic acid samples (for example human tissue/cell samples) which can include a test (or candidate) sample and a reference (or normal) sample.

In various embodiments, genomic variants can be detected using a nucleic acid sequencing system and/or analysis of sequencing data. The sequencing workflow can begin with the test sample being sheared or digested into hundreds, thousands or millions of smaller fragments which are sequenced on a nucleic acid sequencer to provide hundreds, thousands or millions of nucleic acid sequence reads (i.e., sequence reads). Each read can then be mapped to a reference or target genome, and in the case of mate-pair fragments, the reads can be paired thereby allowing interrogation of repetitive regions of the genome. The results of mapping and pairing can be used as input for various standalone or integrated genome variant (e.g., SNP, CNV, Indel, inversion, etc.) analysis tools.

When genome variants are initially identified in nucleic acid samples, especially during analysis of disease-associated genes, their functional implications might not be immediately evident. Distinguishing between a genomic variant that changes the phenotype and one that does not is a difficult task. An increasing amount of evidence indicates that genomic variants in both coding and non-coding sequences can have unexpected deleterious effects on the splicing of a gene transcript. This makes distinguishing between benign polymorphisms and disease-associated splicing mutations difficult. Therefore, the ability to link the genetic variants identified in a nucleic acid sequence to various pieces of relevant biological information can greatly assist in the determination of the biological significance of the identified genetic variants.

The phrase "functional annotation" denotes data and information that can be relevant to the role that a called variant plays in gene/transcript/protein level function.

The phrase "coding region" denotes the portion of a gene's DNA or RNA, composed of exons that codes for protein. It should be understood, however, that the coding region of mRNA does not typically include the first part of the first exon (the 5' untranslated region) or the last part of the last exon (the 3' untranslated region).

The phrase "intragenic region," "intronic region," or "intron" denotes any nucleotide sequence within a gene that is removed by RNA splicing to generate the final mature RNA product of a gene.

The phrase "intergenic region" denotes a stretch of DNA sequences located between genes that contain few or no genes.

The phrase "sample genome" can denote a whole or partial genome of an organism.

The techniques of "paired-end," "pairwise," "paired tag," or "mate pair" sequencing are generally known in the art of molecular biology (Siegel A. F. et al., Genomics. 2000, 68: 237-246; Roach J. C. et al., Genomics. 1995, 26: 345-353). These sequencing techniques can allow the determination of multiple "reads" of sequence, each from a different place on a single polynucleotide. Typically, the distance (i.e., insert region) between the two reads or other information regarding a relationship between the reads is known. In some situations, these sequencing techniques provide more information than does sequencing two stretches of nucleic acid sequences in a random fashion. With the use of appropriate software tools for the assembly of sequence information (e.g., Millikin S. C. et al., Genome Res. 2003, 13: 81-90; Kent, W.J. et al., Genome Res. 2001, 11: 1541-8) it is possible to make use of the knowledge that the "paired-end," "pairwise," "paired tag" or "mate pair" sequences are not completely random, but are known to occur a known distance apart and/or to have some other relationship, and are therefore linked or paired in the genome. This information can aid in the assembly of whole nucleic acid sequences into a consensus sequence.

COMPUTER-IMPLEMENTED SYSTEM

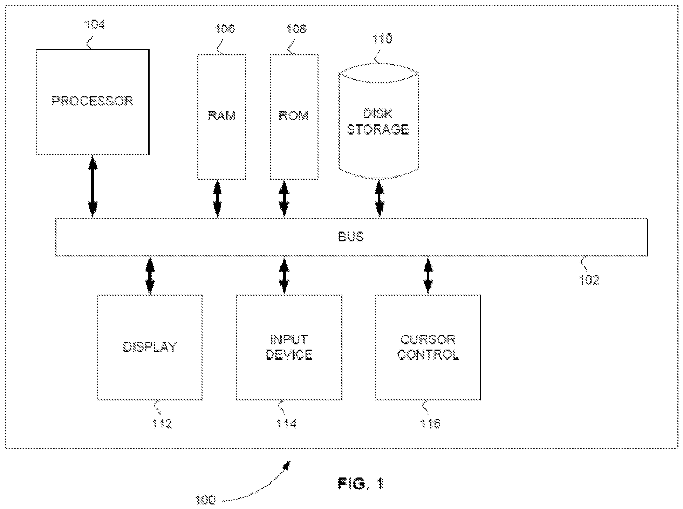

Figure 1 is a block diagram that illustrates a computer system 100, upon which embodiments of the present teachings may be implemented. In various embodiments, computer system 100 can include a bus 102 or other communication mechanism for communicating information, and a processor 104 coupled with bus 102 for processing information. In various embodiments, computer system 100 can also include a memory 106, which can be a random access memory (RAM) or other dynamic storage device, coupled to bus 102 for determining base calls, and instructions to be executed by processor 104. Memory 106 also can be used for storing temporary variables or other intermediate information during execution of instructions to be executed by processor 104. In various embodiments, computer system 100 can further include a read only memory (ROM) 108 or other static storage device coupled to bus 102 for storing static information and instructions for processor 104. A storage device 110, such as a magnetic disk or optical disk, can be provided and coupled to bus 102 for storing information and instructions.

In various embodiments, computer system 100 can be coupled via bus 102 to a display 112, such as a cathode ray tube (CRT) or liquid crystal display (LCD), for displaying information to a computer user. An input device 114, including alphanumeric and other keys, can be coupled to bus 102 for communicating information and command selections to processor 104. Another type of user input device is a cursor control 116, such as a mouse, a trackball or cursor direction keys for communicating direction information and command selections to processor 104 and for controlling cursor movement on display 112. This input device typically has two degrees of freedom in two axes, a first axis (i.e., x) and a second axis (i.e., y), that allows the device to specify positions in a plane.

A computer system 100 can perform the present teachings. Consistent with certain implementations of the present teachings, results can be provided by computer system 100 in response to processor 104 executing one or more sequences of one or more instructions contained in memory 106. Such instructions can be read into memory 106 from another computer-readable medium or computer-readable storage medium, such as storage device 110. Execution of the sequences of instructions contained in memory 106 can cause processor 104 to perform the processes described herein. Alternatively hard-wired circuitry can be used in place of or in combination with software instructions to implement the present teachings. Thus implementations of the present teachings are not limited to any specific combination of hardware circuitry and software.

The term "computer-readable medium" (e.g., data store, data storage, etc.) or "computer-readable storage medium" as used herein refers to any media that participates in providing instructions to processor 104 for execution. Such a medium can take many forms, including but not limited to, non-volatile media, volatile media, and transmission media. Examples of non-volatile media can include, but are not limited to, optical or magnetic disks, such as storage device 110. Examples of volatile media can include, but are not limited to, dynamic memory, such as memory 106. Examples of transmission media can include, but are not limited to, coaxial cables, copper wire, and fiber optics, including the wires that comprise bus 102.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

Various forms of computer readable media can be involved in carrying one or more sequences of one or more instructions to processor 104 for execution. For example, the instructions can initially be carried on the magnetic disk of a remote computer. The remote computer can load the instructions into its dynamic memory and send the instructions over a telephone line using a modem. A modem local to computer system 100 can receive the data on the telephone line and use an infra-red transmitter to convert the data to an infra-red signal. An infra-red detector coupled to bus 102 can receive the data carried in the infra-red signal and place the data on bus 102. Bus 102 can carry the data to memory 106, from which processor 104 retrieves and executes the instructions. The instructions received by memory 106 may optionally be stored on storage device 110 either before or after execution by processor 104.

In accordance with various embodiments, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

## NUCLEIC ACID SEQUENCING PLATFORMS

Nucleic acid sequence data can be generated using various techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

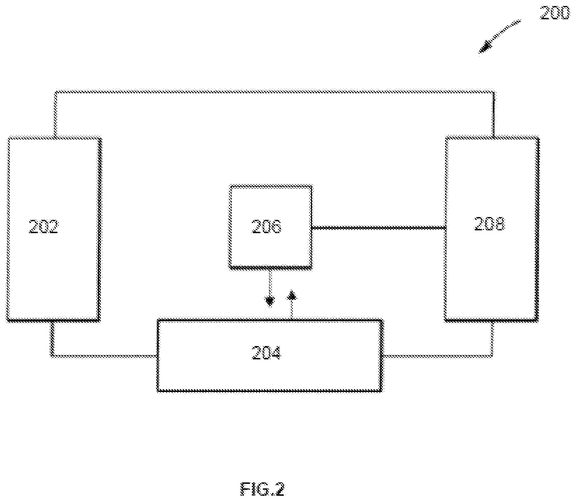

Various embodiments of nucleic acid sequencing platforms (i.e., nucleic acid sequencer) can include components as displayed in the block diagram of Figure 2. According to various embodiments, sequencing instrument 200 can include a fluidic delivery and control unit 202, a sample processing unit 204, a signal detection unit 206, and a data acquisition, analysis and control unit 208. Various embodiments of instrumentation, reagents, libraries and methods used for next generation sequencing are described in U.S. Patent Application Publication No. 2007/066931(ASN 11/737308) and U.S. Patent Application Publication No. 2008/003571 (ASN 11/345,979) to McKernan, et al.

In various embodiments, the fluidics delivery and control unit 202 can include reagent delivery system. The reagent delivery system can include a reagent reservoir for the storage of various reagents. The reagents can include RNA-based primers, forward/reverse DNA primers, oligonucleotide mixtures for ligation sequencing, nucleotide mixtures for sequencing-by-synthesis, optional ECC oligonucleotide mixtures, buffers, wash reagents, blocking reagent, stripping reagents, and the like. Additionally, the reagent delivery system can include a pipetting system or a continuous flow system which connects the sample processing unit with the reagent reservoir.

In various embodiments, the sample processing unit 204 can include a sample chamber, such as flow cell, a substrate, a micro-array, a multi-well tray, or the like. The sample processing unit 204 can include multiple lanes, multiple channels, multiple wells, or other means of processing multiple sample sets substantially simultaneously. Additionally, the sample processing unit can include multiple sample chambers to enable processing of multiple runs simultaneously. In particular embodiments, the system can perform signal detection on one sample chamber while substantially simultaneously processing another sample chamber. Additionally, the sample processing unit can include an automation system for moving or manipulating the sample chamber.

In various embodiments, the signal detection unit 206 can include an imaging or detection sensor. For example, the imaging or detection sensor can include a CCD, a CMOS, an ion sensor, such as an ion sensitive layer overlying a CMOS, a current detector, or the like. The signal detection unit 206 can include an excitation system to cause a probe, such as a fluorescent dye, to emit a signal. The expectation system can include an illumination source, such as arc lamp, a laser, a light emitting diode (LED), or the like. In particular embodiments, the signal detection unit 206 can include optics for the transmission of light from an illumination source to the sample or from the sample to the imaging or detection sensor. Alternatively, the signal detection unit 206 may not include an illumination source, such as for example, when a signal is produced spontaneously as a result of a sequencing reaction. For example, a signal can be produced by the interaction of a released moiety, such as a released ion interacting with an ion sensitive layer, or a pyrophosphate reacting with an enzyme or other catalyst to produce a chemiluminescent signal. In another example, changes in an electrical current can be detected as a nucleic acid passes through a nanopore without the need for an illumination source.

In various embodiments, data acquisition analysis and control unit 208 can monitor various system parameters. The system parameters can include temperature of various portions of instrument 200, such as sample processing unit or reagent reservoirs, volumes of various reagents, the status of various system subcomponents, such as a manipulator, a stepper motor, a pump, or the like, or any combination thereof.

It will be appreciated by one skilled in the art that various embodiments of instrument 200 can be used to practice variety of sequencing methods including ligation-based methods, sequencing by synthesis, single molecule methods, nanopore sequencing, and other sequencing techniques. Ligation sequencing can include single ligation techniques, or change ligation techniques where multiple ligation are performed in sequence on a single primary nucleic acid sequence strand. Sequencing by synthesis can include the incorporation of dye labeled nucleotides, chain termination, ion/proton sequencing, pyrophosphate sequencing, or the like. Single molecule techniques can include continuous sequencing, where the identity of the nuclear type is determined during incorporation without the need to pause or delay the sequencing reaction, or staggered sequence, where the sequencing reactions is paused to determine the identity of the incorporated nucleotide.

In various embodiments, the sequencing instrument 200 can determine the sequence of a nucleic acid, such as a polynucleotide or an oligonucleotide. The nucleic acid can include DNA or RNA, and can be single stranded, such as ssDNA and RNA, or double stranded, such as dsDNA or a RNA/cDNA pair. In various embodiments, the nucleic acid can include or be derived from a fragment library, a mate pair library, a chromatin immuno-precipitation (ChIP) fragment, or the like. In particular embodiments, the sequencing instrument 200 can obtain the sequence information from a single nucleic acid molecule or from a group of substantially identical nucleic acid molecules.

In various embodiments, sequencing instrument 200 can output nucleic acid sequencing read data in a variety of different output data file types/formats, including, but not limited to: *.fasta, *.csfasta, *.xsq, *seq.txt, *qseq.txt, *.fastq, *.sff, *prb.txt, *.sms, *srs and/or *.qv.

## GENE EXPRESSION CLASSIFIER

Systems and methods for identifying critical Gene Signatures (Patient Disease Class Discovery) and predicting responders (Patient Treatment Class Prediction) to medical treatments using nucleic acid sequence data, are disclosed.

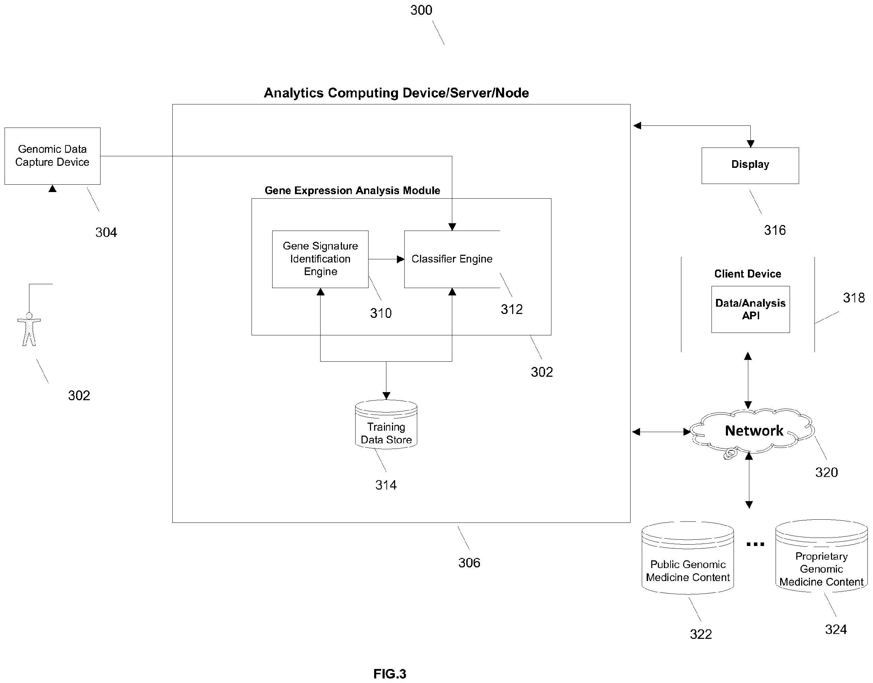

Figure 3 is a schematic diagram of a system for predicting patient response to targeted therapies, in accordance with various embodiments. As depicted herein, the system 300 can include a genomic data capture device 304 (e.g., nucleic acid sequencer, real-time/digital/quantitative PCR instrument, microarray scanner, etc.), an analytics computing device/server/node 306, a display 316 and/or a client device terminal 318, and one or more public 322 and proprietary 324 genomic medicine content sources.

In various embodiments, the analytics computing sever/node/device 302 can be communicatively connected to the genomic data capture device 304, client device terminal 318, public genomic medicine content source 322 and/or proprietary genomic medicine content source 324 via a network connection 320 that can be either a "hardwired" physical network connection (e.g., Internet, LAN, WAN, VPN, etc.) or a wireless network connection (e.g., Wi-Fi, WLAN, etc.).

In various embodiments, the analytics computing device/server/node 302 can be a workstation, mainframe computer, distributed computing node (part of a "cloud computing" or distributed networking system), personal computer, mobile device, etc. In various embodiments, the genomic data capture device 304 can be a nucleic acid sequencer, real-time/digital/quantitative PCR instrument, microarray scanner, etc. It should be understood, however, that the genomic data capture device 304 can essentially be any type of instrument that can generate nucleic acid sequence data from samples obtained from an individual 306.

The analytics computing server/node/device 302 can be configured to host a gene expression analysis module 302 (i.e. Gene Expression Classifier) and a training data store 314. The gene expression analysis module 302 includes a gene signature identification engine 310 and a classifier engine 312 that can be communicatively connected to or networked with each other and the training data store 314.

The training data store 314 is configured to store one or more historical patient feature data records. Historical patient feature data records include information relating to a patient's gene expression profile and medical/health history. In some embodiments, historical patient feature data records further include demographics and/or personal and lifestyle characteristics. In various embodiments, the training data store 314 is resident (stored) in the same computing device as the gene expression analysis module 302. In various embodiments, the training data store 314 is resident in a separate computing device than the gene expression analysis module 302.

The gene signature identification engine 310 is configured to receive historical patient feature data records from the training data store 314 and identify medically and therapeutically significant genomic signatures that are correlated to particular medical conditions and relevant to predicting a success parameter for a particular therapeutic regimen associated with the medical condition by applying a Principal Component Analysis (PCA) algorithm to the historical patient feature records. In an alternative embodiment, the gene signature identification engine 310 utilizes a modified sparse Principal Component Analysis (PCA) algorithm. Principal Component Analysis (PCA) is a mathematical procedure that converts a set of correlated observations into component parts that are independent of each other. The analysis uses eigenvector/eigenvalue decomposition or Singular Value Decomposition (SVD) as the underlying mathematical engine to decompose the observations into its underlying independent components. At the risk of being overly simplifying, we can think of our sense taste as being able to perform something akin to a PCA when we eat food. It decomposes the taste of what we eat into (at least?) 5 independent taste sensations ("sweet", "bitter", "sour", "salty" and "umami"). Sugar, for instance, has "sweet" as the dominant "principal component". Chocolate typically has "sweet" as the dominant "principal component" and "bitter" as the second "principal component." Additionally, certain foods and seasonings consist of combinations of the basic independent tastes that are independent of each other. As a result a skilled chef can identify/guess the approximate amount of ingredients like salt, sugar, curry, pepper, etc that went into dish by tasting it, especially if they are given a list of ingredients in the dish. These ingredients can be thought of as being near orthogonal (independent) tasting "principal components." The PCA process mathematically performs what our sense of taste is approximately performing.

The classifier engine 312 can be configured to receive a genomic signature and a proposed therapeutic regimen (e.g., drug therapies, surgical options, lifestyle/behavioral modifications, etc.) for a prospective patient, associate the genomic signature with the therapeutically significant genomic signatures identified by the gene signature identification engine 310, and generate a predicted success parameter for the proposed therapeutic regimen based on the genomic signature of the prospective patient using an eigen-based classification algorithm. This Eigen-based classification algorithm uses eigenvector/eigenvalue decomposition as the underlying mathematical engine to decompose the observations into its underlying independent components. At the risk of being too simplifying, the eigenvector associated with a set of correlated data or observations can be thought of as one of the independent components that make up the data. That is the correlated data can be decomposed into these independent components. In the case of our previous example of tasting a dish, an eigenvector would correspond to one of the basic tastes ("sweet") or corresponding taste proxies (sugar). The eigenvalue would correspond to the strength of that basic taste/taste proxy in the dish. Eigenvector/eigenvalue is a mathematical method to perform this kind of decomposition of data into independent components.

In various embodiments, the prospective patient's genomic signature is acquired using a genomic data capture device 304.

The success parameter is a predicted cure rate, reduction of disease progression, or patient lifespan increase for the prospective patient being treated with the proposed therapeutic regimen.

Client terminal 320 can be a thin client or thick client computing device. In various embodiments, client terminal 320 can have a web browser (e.g., INTERNET EXPLORER™, FIREFOX™, SAFARI™, etc) that can be used to communicate information to and/or control the operation of the gene signature identification engine 310 and a classifier engine 312 and training data store 314 using a browser to control their function. For example, the client terminal 320 can be used to configure the operating parameters (e.g., predicted success parameters, filtering parameters, data security and retention parameters, etc.) of the various modules, depending on the requirements of the particular application. Similarly, client terminal 320 can also be configure to display the results of the analysis performed by the gene expression analysis module 306 and the gene data capture device 304.

It should be understood that the various data stores disclosed as part of system 300 can represent hardware-based storage devices (e.g., hard drive, flash memory, RAM, ROM, network attached storage, etc.) or instantiations of a database stored on a standalone or networked computing device(s).

It should also be appreciated that the various data stores and modules/engines shown as being part of the system 300 can be combined or collapsed into a single module/engine/data store, depending on the requirements of the particular application or system architecture. Moreover, in various embodiments, the system 300 can comprise additional modules, engines, components or data stores as needed by the particular application or system architecture.

In various embodiments, the system 300 can be configured to process the nucleic acid reads in color space. In various embodiments, system 300 can be configured to process the nucleic acid reads in base space. In various embodiments, system 300 can be configured to process the nucleic acid sequence reads in flow space. It should be understood, however, that the system 300 disclosed herein can process or analyze nucleic acid sequence data in any schema or format as long as the schema or format can convey the base identity and position of the nucleic acid sequence.

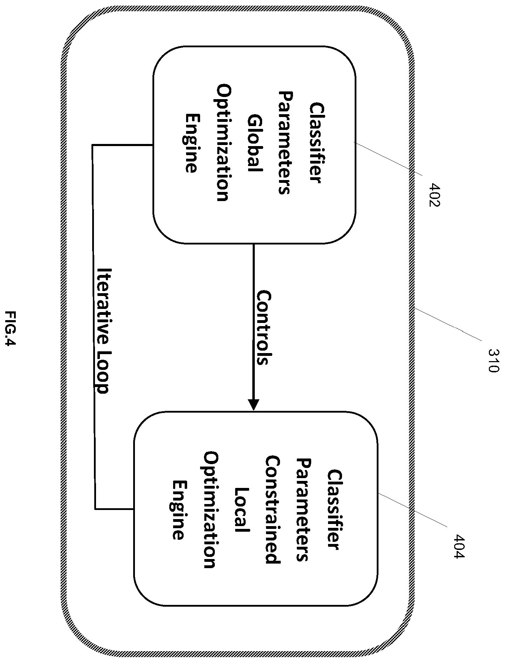

Figure 4 is a schematic diagram detailing the operation of the gene signature identification engine, in accordance with various embodiments.

As depicted herein, in various embodiments, the gene signature identification engine 310 uses global 402 and constrained local 404 classifier parameter optimization engines to control how it determines the classifier parameters used by the classifier engine's 312 special Eigen-based inversion algorithm to calculate a patient's expected clinical response. That is, the global optimization engine 402 and the constrained local optimization engine 404 are configured to perform parameterized re-scalings, biasing, and normalizations of the historical patient feature records or "training data" (input data) to transform it from a domain-specific "data space" into a less domain-specific "information space." These data-transformation parameters can be referred to as the "classifier parameters." In various embodiments, 4 or less classifier parameters are required to characterize the classifier system. In a preferred embodiment, just two classifier parameters are used to characterize the classifier system. This represents a low-order parameter characterization of the system. The global 402 and constrained local 404 classifier parameter optimization engines are used within the gene signature identification engine 310 to determine good values for the data-transformation classifier parameters. Imagine an extremely rough bumpy hilly (2-dimensional) terrain and imagine yourself as a mountain climber (i.e. a local optimizer) determined to climb the top of the nearest hill/mountain. If you also know, a priori, that the hilltops are located within a well defined terrain, you would want to constrain the paths that you as the mountaineer take to find the nearest mountaintop to be within this terrain. This is what the local constrained optimization engine does mathematically. However, it should be clear that where you start would determine which local maxima (hilltop or mountaintop) that you reach. A different starting location can lead to a different hilltop. For a general terrain, there is no way to make a typical local optimization engine find the highest mountain in the terrain. That's where global optimization comes in. The idea is to create a process (search heuristic) to generate a variety of starting points then pick the highest hill reaches as the best guess for the highest hill/mountain in the terrain. This process is called global optimization. A Genetic Algorithm (GA) is a global search heuristic that attempts to mimic the process of natural evolution to generate a better set of starting search locations than would be generated just randomly. The GA uses inheritance, mutation, survival of the fittest, and other evolutionary ideas to generate new ("child") starting locations from prior ("parent") starting locations. The starting set of parameters that lead to the maximum value of the objective function (i.e. the highest mountain) using the combined global and constrained local optimization engine components of the Gene Signature Identification algorithm would then be accepted as the "best" parameter set.

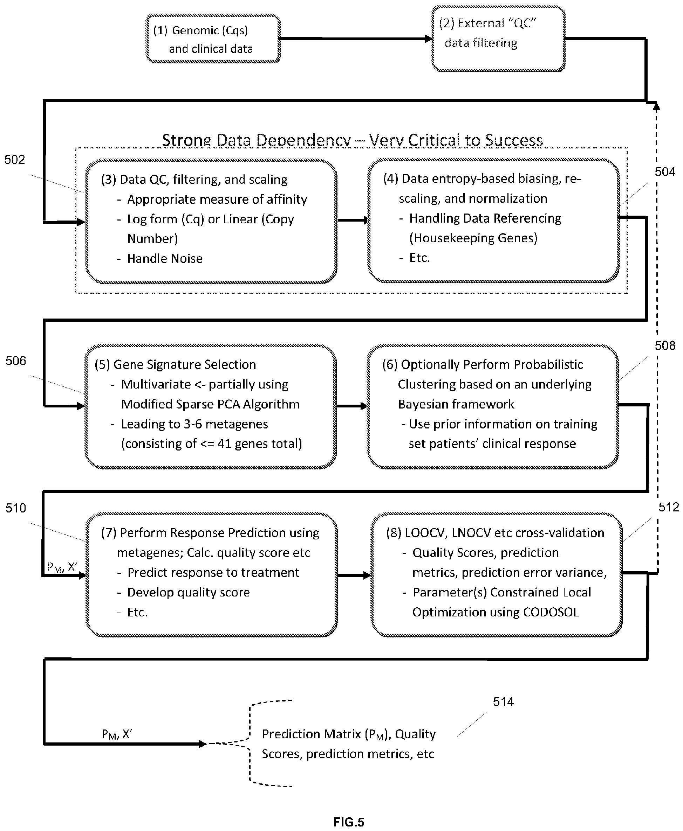

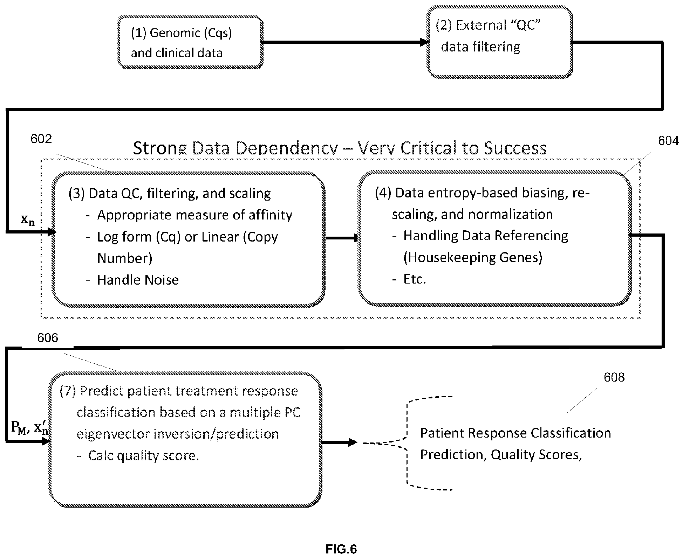

Figures 5 and 6 are exemplary flowcharts showing a method for predicting patient response to targeted therapies, in accordance with various embodiments.

As depicted herein, steps 502 thru 508 details the analytics workflow utilized by the gene signature identification engine 310 to identify therapeutically significant gene signatures from a training data set (input data) containing historical patient feature including gene expression profile and medical/health history data records, and further including genomic profile (e.g., biomarker), patient demographics, and/or personal and lifestyle characteristics data records. Specifically, in steps 502 and 504, the gene signature identification engine 310 utilizes a parameterized series of scaling and normalization weights to convert the input data from a domain-specific "data space" into a less domain-specific "information space." In step 506, the gene signature identification engine 310 performs multivariate gene signature identification using a specially developed algorithm based on a weighted sparse PCA (Principal Component Analysis) to identify therapeutically significant gene signatures. In step 508, the gene signature identification engine 310 can optionally perform an optimization operation to adaptively find local optimizations of genomic and clinical response algorithm parameter normalization values and patient weightings. In step 510, the gene signature identification engine 310 generates therapeutic response predictions (i.e., predicted success parameters of one or more therapeutic regimen) for the previously identified therapeutically significant gene signatures. This step can also result in the generation of a quality score for each identified therapeutically relevant gene signature. The success parameter is a predicted cure rate, reduction of disease progression, or patient lifespan increase for a patient being treated with a particular therapeutic regimen.

In step 512, the gene signature identification engine 310 uses a Genetic Algorithm (GA) to perform global optimization using the algorithm parameters as genes to superimpose a more complete global optimization methodology using the local optimization methodology. The combined usage of the global optimization (GA) methodology using the constrained local optimization methodology to lead to estimates of the algorithm parameters that lead to better estimates of Gene Signatures and Patient Treatment Response predictions was explained earlier. This step can also result in the generation of therapeutic regimen prediction metrics, prediction error values, etc.

As depicted herein, steps 602 thru 608 details the analytics workflow utilized by the classifier engine 312 to predict patient response to targeted therapies. In steps 602 and 604, the classifier engine 312 utilizes a parameterized series of scaling and normalization weights to convert a prospective patient's gene signature (i.e., input data) from a domain-specific "data space" into a less domain-specific "information space." In step 606, the classifier engine 312 generates therapeutic response predictions (i.e., predicted success parameters for the proposed therapeutic regimen) by applying an Eigen-based inversion algorithm to one or more proposed therapeutic regimen and the gene signature data obtained from a prospective patient. In various embodiments, the success parameter is a predicted cure rate, reduction of disease progression, or patient lifespan increase for the prospective patient being treated with the proposed therapeutic regimen.

In step 608, the classifier engine 312 generates prediction metrics and quality scores for the therapeutic response predictions.

## DETAILED DISCUSSION

In order to fully explain the various alternative embodiments, we will make the following definitions:

$$N \equiv The\ total\ number\ of\ patients\ in\ the\ training\ set \qquad (1)$$

$$P \equiv The\ total\ number\ of\ patient\ Gx, non-Gx, and\ clinical\ response\ data\ \text{types} \qquad (2)$$

The original data vector associated with each patient will consist of gene expression data (e.g. characterized by Cq) **plus** other known patient data like demographic, lifestyle, and medical history data **plus** meaningful (and appropriately scaled) clinical response data. This clinical response data can be either binary (responder/non-responder), or real (e.g. survival time) data. The weighting factor used to scale the data will be appropriate to scale the total contribution of that clinical data term to the overall eigenvector calculation re the gene expression information.

Thus, P represents the total number of the patient's genes, demographic data types, and/or other patient data types plus the number of the patient's clinical response data types.

$$P_{Known} \equiv \text{The total number of always known patient data types such as } Gx \text{ and known non} - Gx \text{ data} \tag{3}$$

$$P_{Pred} \equiv \text{The total number of the types of patient's data that needs to be predicted outside of the training dataset such as the clinical response data types} \tag{4}$$

Thus, it's clear that

$$P = P_{Known} + P_{Pred}. \tag{5}$$

We will assume, for now, that we've already transformed the original patient Training Set data via re-scaling, normalization and biasing to produce a matrix, X, containing the patients' transformed quantitative data. This transformation process can be thought of as transforming the data from a domain-specific data space to a less domain-specific information space. We can define this normalized and transformed patient matrix as:

$$X\,[P \times N] \equiv \begin{bmatrix} [X_{Pred}] \\ [X_{Known}] \end{bmatrix} \tag{6}$$

where,

$$X_{Known}[P_{Known} \times N] \equiv [\text{Matrix with the known Gx and other Patient Data}], \tag{7}$$

and

$$X_{Pred}[P_{pred} \times N] \equiv [\text{Matrix with the Patient Clinical Response Data that needs to be predicted}] \tag{8}$$

We will define the patients' features symmetric cross-correlation matrix as the matrix multiplication of X and its transpose such that

$$C[P \times P] \equiv XX^T, \tag{9}$$

where, as is standard, the transpose of a matrix is indicated by a superscripted T. Each row of C represents the cross-correlations corresponding to features in the corresponding row number of X. Since C is symmetric, each column of C represents the cross-correlations corresponding to one of the P features in the corresponding row number of X. Without loss of generality, X is typically normalized so that the diagonal elements of the matrix C are all ones. That is each row of X is perfectly correlated with itself. The resulting non-diagonal elements of the matrix C have magnitudes that are always $\leq 1$.

The standard PCA (Principal Component Analysis) approach is to first solve the eigenproblem

$$CR \equiv R\Lambda \tag{10}$$

to get the full $[P \times P]$ (right) eigenvector matrix R and its corresponding $[P \times P]$ diagonal eigenvalue matrix $\Lambda$. The diagonal eigenvalue matrix $\Lambda$ has the generally non-zero eigenvalues along its diagonal.

Each column of R represents a different eigenvector. These eigenvectors are, in general, "full' vectors. That is each of its rows are typically significantly non-zero. Each row of the eigenvector matrix C corresponds to one of the P features in the corresponding row number of X.

Since the cross-correlation matrix C is symmetric, its associated right eigenvectors (the columns of R) are orthogonal. As a result of this orthogonality,

$$R^T R = I, \tag{11}$$

where, as is standard, I is defined to the diagonal identity matrix with ones along its diagonal and zeros elsewhere.

The Principal Component is defined to be the eigenvector corresponding to the largest eigenvalue in A. That is, it is the column R corresponding to the column of A with the largest diagonal (eigenvalue) element. It is generally true that the eigenvectors corresponding to the largest eigenvalues is most likely to capture true informational patterns in the patient data matrix X and its associated cross-correlation matrix C. As a result, the Principal Component eigenvector generally contains the information. On the other hand, the eigenvectors corresponding to the smallest eigenvalues represents idiosyncratic noise in the patient data matrix X and its associated cross-correlation matrix C. The dividing line that separates which eigenvectors represent noise and which ones corresponds to true informational patterns is typically not known apriori. It can be very data dependent. It typically requires special methods to decide which eigenvectors represent "noise" and which ones represent "information". The disclosed concepts allow us to make this separation in a very clear manner. Partially as a result of this difficulty, the typical PCA approach is to only use the Principal Component eigenvector as the basis of the response prediction. However, approaches that do this are probably not using all of the information that could be wrung from the patterns in the patient data matrix X and its associated cross-correlation matrix C. All things being equal, classifiers which use more of the information available in the patterns in the patient data matrix X and its associated cross-correlation matrix C, and are able to ignore the noise, will be more effective than classifiers that use less information. Being able to separate information from noise is a significant challenge for PCA-based approaches.

As noted before, all the eigenvectors of C are "full", i.e. almost all of their elements are non-zero. As a result, identifying the significant genes that should be used to form a gene signature is non-trivial. This is because the eigenvectors, with their many non-zero elements, require contributions from almost ALL the genes available. It is not a satisfactory solution to simply ignore the genes associated with the smallest elements in the Principal Component eigenvector. This is because those genes may be significant ones in other eigenvectors and may be associated with associated with alternative gene pathways.

Similarly, the traditional approach of performing uni-variate statistical p-tests for the significance of the correlation between the expression of the gene and patient response is also less than satisfactory. This method is liable to select a set of genes that are individually strongly correlated with patient response. However, in potentially having gene expressions that are highly correlated with each other, each additional gene isn't really adding much new information to the classification system. Ironically, it may be the genes that are rejected by the uni-variate p-tests as being not significant enough that might have added significant new information to aid the classification system.

Because it is important that the selection of genes for a gene signature be done in a multi-variate manner where the effects and interactions of multiple genes are considered simultaneously, the method to identify the gene signature disclosed herein is multivariate in nature.

**Gene Signature Selection**

The new classifier approach being suggested here uses a modification of the relatively new sparse Principal Component Analysis or sparse PCA algorithm to identify a good gene signature set. The most significant principal component eigenvectors would be used to identify the Gene Signatures ("metagenes"). As before, for the training set, there are P genes, and N patients. Without loss of generality, we're assuming that the classification system is only using gene expression to make its classifications.

In one extreme case, if all of the gene expressions are nearly perfectly correlated with each other, then effectively just a single eigenvector will be required to explain any clinically significant patient information. This single eigenvector will contain contributions from all the genes. This problem will have 1 non-zero eigenvalue and (P - 1) eigenvalues that are zero. Thus, the PCA has reduced the dimensionality of the classification problem from P to 1. There was a lot of redundant information in the gene expression data that could be used to reduce noise. Alternatively, this case can be thought of as illustrating a situation where the original gene set was too narrowly chosen since all of the chosen genes are perfectly correlated with each other. Most gene expression pathways involve multiple genes. It is therefore more likely that the original gene set was too large and/or was incorrectly chosen and contains a lot of genes that are unrelated to each other. Many genes involved in gene expression pathways were not captured by the original gene set and there may be other gene expression pathways that weren't captured by the original gene set.

In another extreme case, assuming all of the gene expressions are nearly perfectly uncorrelated with each other, then approximately P eigenvectors will be required to explain any clinically significant patient information. Thus, the PCA has not reduced the original dimensionality of the classification problem. This could indicate that the original gene set was perfectly chosen and each gene expression contains all of the information to explain clinical patient variation.

In general, if the original gene set is full and/or is carefully chosen, it will include many genes involved in multiple pathways. As such there will be built-in redundancy in gene expression information and the effective dimensionality of the classification can be reduced from P to some number greater than 1.

When using classic PCA, the resulting orthogonal principal component eigenvectors will, typically, have both positive and negative components. This can be interpreted as indicating the relative (in some sense) expression levels of the genes associated with each eigenvector position. However, how are the negative eigenvector components produced by classic PCA to be interpreted in terms of forming the associated metagenes? Clearly, genes should either contribute or not contribute in a purely additive sense to a metagene grouping. Thus, negative PCA eigenvector components are a problem. One approach is to ignore any gene that has a negative eigenvector component. This is clearly sub-optimal. It also makes the resulting eigenvectors non-orthogonal. It has lost information on some gene expression pathways.

One way to resolve this issue is to explicitly constrain the PCA eigenvectors to be non-negative. Alternative non-negative (non-PCA) approaches to matrix factorizations can also be used to come up with non-negative eigenvectors. This leads to principal component eigenvectors that only have non-negative components. No gene represented by the non-negative eigenvector will have a corresponding negative component. Thus, each gene's contribution to a metagene is additive. In doing so, the resulting non-negative eigenvectors become non-orthogonal. They should, however, be nearly orthogonal. Each metagene no longer contains independent information. An additional benefit is that the resulting eigenvectors tend to be more sparse or compact than those resulting from standard PCA.

Although traditional or classic PCA, based on the full eigenvectors, allows minimal loss of information, uncorrelated structures, and relatively easy interpretation of the linear combinations, traditional PCA (especially for high dimensional under-determined applications, such as Gx classifier applications) results in extracted components that are linear combinations of all input variables and can be strongly influenced by noise and outliers.

As noted before, traditional PCA eigenvectors are non-sparse, i.e. all the terms in the PCA eigenvectors are usually non-zero. In effect, creating the full eigenvectors, the classic PCA eigen-analysis attempts to account for, and "explain", data that are "obvious" outliers. The outliers influence all aspects of the analysis, including the Principal Component eigenvectors.

Lack of sparseness is potentially another issue with traditional PCA. Compact eigenvectors means that the number of genes required to form the corresponding metagene sets are smaller. This results in a smaller dictionary of genes that need to be identified/or quantified. Given that sparseness (compactness) is advantageous when it comes to eigenvectors, Explicit derivation of a means to control the sparseness of the eigenvectors would be beneficial.

One way to do this is to add a sparseness term which penalizes "eigenvectors" containing too many non-zero terms (i.e. are not sparse). The extent of this penalization is determined by the size of the sparseness term. The introduction of a non-sparseness penalty causes the resultant eigenvectors to be non-orthogonal. That is each eigenvector may contain some redundant information. This is a minor disadvantage compared to the advantages of sparseness.

It is proposed here to use sparse PCA as the basis of finding metagenes. A critical component of this methodology is that the sparseness of the discovered eigenvectors can be algorithmically controlled by a sparseness parameter. A sparse PCA algorithm was modified to create a sparseness parameter that either controls the maximum number of non-zero elements in each sparse eigenvector or puts a minimum threshold on its non-zero elements.

Many sparsing methods have been proposed that attempt maintain the interpretability of PCA. However, many of these methods tend to suffer from the influence of outlier noise present in the data. Sparse PCA by Iterative Elimination Algorithm is a good modern improvement on the early sparse PCA algorithms. Traditional methods of choosing the genes used in a Gene Signature rely on an ad-hoc mix of uni-variate statistical tools and complicated genes pathway analysis coupled with the investigators' judgment, based on their knowledge and experience. Previous uses of the sparse PCA algorithm, in this and other contexts, concentrated on its ability to filter out the effects of outlier data from an analysis, and were more frequently used in the context of image processing and facial recognition problems. A further advantage of this approach is that we are not using a general sparse PCA algorithm, but that we are using our modification of a Robust PCA algorithm. This relatively new sparse PCA method, called Robust Sparse PCA (RSPCA) method, focuses on enforcing the robustness of the sparse PCA calculation. The robust aspect of this algorithm makes the resulting sparse PCA eigenvectors even more resistant to the effects of outlier data than other sparse PCA algorithms. The robustness of the proposed method to outliers and noises has been supported by a series of experiments performed on the synthetic and face reconstruction problems.

We will define the set of sparse eigenvectors for the correlation matrix C as the matrix $R_s$. The sparse eigenvectors are no longer orthogonal like the full eigenvectors. So that

$$R_S^T R_S \neq I. \tag{12}$$

However, they are "optimally" orthogonal for the level of sparseness requested, and can be near-orthogonal which is defined as

$$R_S^T R_S \approx I. \tag{13}$$

This RSPCA algorithm finds the Principal Components of the feature space where the L1-norm (absolute deviation) dispersion of the input data can be possibly maximized, instead of the L2-norm (root mean square) variance generally employed by the typical sparse PCA methods. In other words, it attempts to minimize the mean of the absolute values of the off-diagonal components of $R_S^T R_S$ in equations 12 and 13. Other sparse PCA methods typically attempt to minimize the square root of the mean of the square (root mean square) of the values of the off-diagonal components of $R_S^T R_S$ in equations 12 and 13.

The RSPCA method only attains an approximation to, but not a strictly rigorous solution to, the original eigenproblem. The orthogonality of the Principal Components calculated by the proposed RSPCA method cannot be mathematically guaranteed and only a local maximum of the L1-norm optimization is strictly guaranteed by the RSPCA method. Orthogonality is a good property. It allows the informational patterns in the correlation matrix to be represented by fewer eigenvectors (compact representation) than with the near-orthogonal sparse eigenvectors. However, the approximation has proven to be sufficient for most real problems. The robustness of the RSPCA method to outliers and noises, relative to traditional PCA and to other popular sparse PCA methods, was demonstrated using series of experiments performed on the synthetic and face reconstruction problems. It was shown that the RSPCA algorithm did a better job, than those other methods, of rejecting the effects of noise and outliers. This is an important property to have for under-determined problems like the Gx Classifier problems. Other similarly effective approaches to robust sparse PCA have been discussed in the literature.

We have modified the RSPCA algorithm to allow us to define the sparseness parameter as a combination of a maximum number of sparse eigenvector elements and a minimum sparse eigenvector element values.

However, because of how the patient data matrix was set up, normalized, and transformed, the associated sparse eigenvectors can be used to identify gene signatures in a multivariate way. As a result, one of the advantages of this methodology is that the resulting correlation matrix should yield some eigenvectors that contain non-zero clinical response terms and others that don't. A key idea is that the eigenvectors that don't contain non-zero clinical response terms can be assumed to be associated with noise. These can be ignored. The eigenvectors that contain non-zero clinical response terms can be assumed to be associated with true metagenes. By adjusting this sparseness parameter using some sort of optimization process (which can be discussed later) we should be able to almost algorithmically identify compacted metagenes. This gives an objective multivariate methodology for identifying metagenes associated with clinical response data.

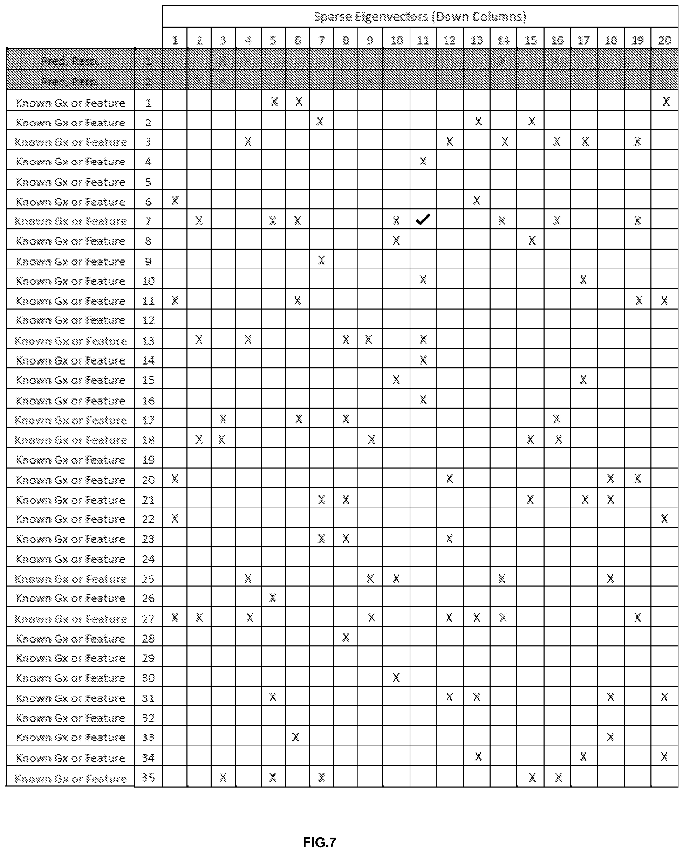

Figure 7 shows a made-up example of a sparse eigenvector matrix $R_S$ containing 35 known features, 2 diagnostic features will need to be predicted outside of the Training Set (rows filled out in blue), and 20 of the sparse eigenvectors with a maximum of 5 elements each. Non-zero elements are marked with an X. All other elements are zero. It is an example of how to use the structure of the elements of a sparse eigenvector matrix $R_s$ to identify Gene Signatures. The 35 known features could be entirely from Gx quantification data or it could also contain other known patient features that have been appropriately scaled, normalized, and biased. This made-up example shows that eigenvectors 2, 3, 4, 9, and 14 contain non-zero elements (marked by the red Xs). The known features 3, 13, 17, 18, 25, 27, and 35 (named in red) are the features contained by these eigenvectors. These set of features (3, 13, 17, 18, 25, 27, and 35) would then be identified as the Gene Signature assuming that the features are made up entirely of Gx quantification data.

Here, the features are being chosen using a multi-variate algorithm that accounts for less frequent but independent information in the patient data (orthogonality).

The Gene Signature identification is done within the getReducedParameterSet engine which implements the following algorithm

1. Take the scaled, normalized, and bias patient data matrix (X).

2. Use a starting input value for the maximum number of sparse eigenvector elements (1/32 number of features could be a good starting point).

3. Set the initial value of the minimum of the sum of the minimum patient response entropies to infinity,

4. Use the input value for the maximum number of sparse eigenvector elements to calculate the robust sparse eigenvectors matrix $R_S$.

5. Calculate the Gene Signature parameter/feature set.

6. Create a smaller updated patient data matrix using only the patients' clinical diagnostic responses/ "prediction" features and the known features corresponding to the Gene Signature parameter/feature set.

7. Calculate an updated weighted correlation matrix corresponding to the smaller updated patient data matrix.

8. Calculate a full updated eigenvector matrix corresponding to the updated weighted correlation matrix.

9. Predict updated scaled patient responses using a scaledPatientResponsePrediction engine to be described later.

10. Send the updated scaled patient responses to the getMinimumPatientResponseEntropy engine (to be described later) to calculate the minimum patient response entropies for the updated scaled patient responses.

11. Sum the minimum patient response entropies for the updated scaled patient responses and compares it with the previous minimum of the sum of the minimum patient response entropies. Set the minimum of the sum the minimum patient response entropies to the current value of the sum the minimum patient response entropies and the "optimum" values for maximum number of sparse eigenvector elements and the "optimum" Gene Signature parameter/feature set to the current values, **if and only** if the current sum the minimum patient response entropies is less than the current minimum of the sum the minimum patient response entropies,

12. Update the input value for the maximum number of sparse eigenvector elements by one, returns to step 4, stopping only when the input value for the maximum number of sparse eigenvector elements reach a maximum value (1/8 number of features could be a good ending value).

Thus, an advantage of various embodiments of this methodology is that the embodiments specify both the algorithm for identifying good Gene Signatures sets and an algorithm for determining the approximate "best" Gene Signature out of a set of possible calculated Gene Signatures using the inherent statistical entropic properties of the predictions from the Gene Signatures.

**Creating the Patient Data Matrix (X) using the createClassifierDataDataMatrix Engine Algorithm**

The elements of an initially formed Patient Data Matrix ($X_0$) needs to be properly scaled, biased, and normalized to transform it into a form that can yield good gene signatures and make good multi-eigenvector patient response predictions.

The creation of the Patient Data Matrix (X) is done within the createClassifierDataDataMatrix engine which implements the following parameterized algorithm to create and transform an initially formed initial Patient Data Matrix ($X_0$) by scaling, biasing, and normalizing it to form the Patient Data Matrix (X):

1. Take certain run parameters which control the scaling, normalization, and (potentially) biasing of the patient data matrix (X).

2. When creating a Patient Data Matrix for Training Set data so that the patients' diagnostic data are available, create **prediction response rows** corresponding to the patients' clinical response data by converting response notations such as CR, PR, SD, MxR, and PD into numerical designations (such as 5, 4, 3, 2, 1).

3. Create some **known non-Gx feature rows** corresponding to the patients' treatment data by converting presence/absence of particular treatments into binary 1/0 or 1/-1 designations.

4. Create some additional **known non-Gx feature rows** corresponding to particular types of patients' features, such as demographic data, by using appropriate numerical codings for non-numerical data (e.g. Female -> 1, and Male -> -1; smoker -> 1 and non-smoker -> -1; strong exerciser -> 1, moderate to occasional exerciser -> 0, and non-exerciser -> -1; etc).

5. Create some additional **known Gx feature rows** corresponding to the patients' Gx quantification data by appending the patients' Gx quantification data.

6. Create normalized known non-Gx feature rows by using some of the run parameters to control the operation of the normalizeNonGxData engine (to be described later),

7. Calculate the "bias" associated with each Gx feature row by running the getPatientResponseEntropy engine (to be described later) to determine the midpoint value for that Gx feature that best separates responders from non-responders from the standpoint of statistical entropy.

8. Create (re)scaled known Gx feature rows by using scaleGxData engine, the previously calculated bias (pcrBias ) and an effective PCR efficiency(effecPCREffic) input run parameter, and the unscaled Gx Data (assumed here to be PCR Gx data) to create the (re)scaled known Gx feature rows.

Here are the re-scaling calculations performed by the scaleGxData engine in a preferred embodiment (described using Matlab coding however the mathematical intent should be clear):

$$\mathrm{pcrGxDataUse} \ = \ (\mathrm{pcrGxData} + \mathrm{pcrBias});$$

$$\mathrm{scaledPCRGxData} = \text{-sign}(\mathrm{pcrGxDataUse}) \ .* \ (1.0 + \mathrm{effecPCREffic}).\verb|^|\mathrm{abs}(\mathrm{pcrGxDataUse});$$

In another alternative embodiment the rescaling becomes:

$$\mathrm{scaledPCRGxData} = \text{-sign}(\mathrm{pcrGxDataUse}) \ .* \ ( \ (1.0 + \mathrm{effecPCREffic}).\verb|^|\mathrm{abs}(\mathrm{pcrGxDataUse}) - 1.0);$$

Other similar types of re-scaling of the Gx data can be envisioned.

9. Create normalized known Gx feature rows by using some of the run parameters to control the operation of the normalizeGxData engine (to be described later).

10. In Training mode, finally create the Training Set Patient Data Matrix by using the getWeightedAndScaledTrainingMatrix engine (to be described later) to perform a parameterized transformation of the prediction response data, then pull together all of the transformed data to form the Patient Data Matrix (X).

11. In non-Training mode (i.e. patient response prediction mode), finally create the Patient Data Matrix by using the getWeightedAndScaledTrainingMatrix engine (to be described later) to pull together all of the transformed (known Gx and non-Gx) data to form the Patient Data Matrix (X).

12. Scale the rows (consisting of PPred rows) of the Patient Data Matrix associated with the patient response prediction so as to increase relative importance of these rows with respect to the other rows associated with known patient characteristics (consisting of PKnown rows) such as Gx. Good scaling factors to use would be sqrt(PKnown) or sqrt(PKnown/PPred), where sqrt(x) is defined to mean the square root of x. That is multiply the rows of the Patient Data Matrix associated with the patient response prediction with this scaling factor, such as sqrt(PKnown) or sqrt(PKnown/PPred).

**The getPatientResponseEntropy Engine Algorithm**

The getPatientResponseEntropy Engine Algorithm calculates the pcrBias estimates and the minimum patient entropies for the Gx values that produce the minimum entropies in separating responders from non-responders, for each Gx feature (gene), based on the Training Set data. The minimum entropy (PCR) Gx value, from the standpoint of uni-variate statistical entropy, represents the Gx value for that Gx feature (gene) which best separates responders from non-responders. This minimum entropy (PCR) Gx value is a key value in the non-linear re-scaling of the Gx data prior to a more traditional linear normalization of the data.
The algorithm is:

1. For the Training Set data, for each Gx feature (gene), sort the patient list from the highest to lowest Gx value.

2. For each Gx feature (gene), starting from the highest patient Gx value, calculate the fractions of responders and non-responders for patients with Gx values less than and greater than or equal to that value.

3. For each Gx feature (gene), use these four calculated fractions to determine the total entropy associated with using that Gx value as a threshold for separating responders from non-responders. The four groups are the responder fractions less than the Gx value, the non- responder fractions less than the Gx value, the responder fractions greater than the Gx value, and the non- responder fractions greater than the Gx value. The total entropy is sum of the entropies for each of the four groups and is given by:

$$-\sum_{n=1}^{4}(groupEntropyFractions_n * log(groupEntropyFractions_n))$$

4. For each Gx feature (gene), determine the Gx value which minimizes the total entropy and the minimum total entropy.

5. For each Gx feature (gene), and based on how the bias is being defined and used, the pcrBias Gx value becomes the negative of the Gx value which minimizes the total entropy for that feature (gene).

**The runClassifierDataAnalysis Engine Algorithm**

The runClassifierDataAnalysis Engine Algorithm takes the set of a patient's known Gx data, plus any other known non-Gx data that was used in the Training set, to calculate their predicted treatment response. That predicted treatment response is then thresholded to classify the patient as either a responder or a non-responder.

The algorithm is:

1. Take the Training Set Patient Data Matrix (X) for the reduced feature parameters set that's based on the Gene Signature.

2. Calculate the weighted correlation matrix (C) given by = $XX^T$.

3. Now solve the full eigenproblem for the weighted correlation matrix for the reduced feature parameters set

$$CR \equiv R\Lambda,$$

to get the eigenvectors and eigenvalues for the weighted correlation matrix for the reduced feature parameters set.

4. Filter the eigenvector set to only contain eigenvectors associated with eigenvalues greater than a certain fraction (e.g. 10%) of the maximum eigenvalue. Identify this filtered eigenvector set as $R_{filt}$ and the diagonal matrix of filtered eigenvalues as $\Lambda_{filt}$.

5. Input the vector of known patient data and the filtered eigenvectors set $R_{filt}$ into the getRespPred Engine Algorithm (described later).

6. Get the transfrormed predicted patient response from the getRespPred Engine Algorithm (described later).

7. Inverse transform the transfrormed predicted patient response to get the predicted patient response.

8. Apply a threshold to the predicted patient response to determine whether the patient is a probable responder or not.

**The getRespPred Engine Algorithm**

The getRespPred Engine Algorithm takes the set of a patient's known Gx data, plus any other known non-Gx data that was used in the Training set and the filtered eigenvector set, $R_{filt}$, to calculate their transformed predicted treatment response. The transformed predicted treatment response needs to be inverse transformed to get the predicted treatment response outside of the getRespPred Engine.

The algorithm is:

1. Define a P $\times$ (P$_{\text{filt}}$ + P$_{\text{pred}}$) matrix $R'_{filt}$ as:

$$R'_{filt} \equiv \left[ R_{filt} \left[ \frac{-I}{0} \right] \right],$$

where I is a P$_{\text{pred}}$ $\times$ P$_{\text{pred}}$ identity matrix and 0 is a ((P - P$_{\text{pred}}$) $\times$ P$_{\text{pred}}$) matrix of zeros.

2. Define a P $\times$ 1 vector $x'_{know}$ as:

$$x'_{know} \equiv \begin{bmatrix} \bar{0} \\ x_{know} \end{bmatrix},$$

where $\bar{0}$ is a (P$_{\text{pred}}$ $\times$ 1) vector of zeros and $x_{know}$ is a vector with the known patient information.

3. Calculate $\alpha$, a vector with a set of prediction parameters as:

$$\alpha = pinv\left( \left(R'_{filt}\right)^T R'_{filt} \right) \left(R'_{filt}\right)^T x'_{know},$$

where the notation $pinv\left( \left(R'_{filt}\right)^T R'_{filt} \right)$ represents the pseudo-inverse of the matrix

$$\left(R'_{filt}\right)^T R'_{filt}.$$

4. Identify the transformed predicted treatment response as the last P$_{\text{pred}}$ elements of the vector $\alpha$.

**The normalizeNonGxData Engine Algorithm**

The normalizeNonGxData Engine Algorithm takes the set of a patient's known non-Gx data and normalizes it.
The algorithm is:

1. For each non-Gx feature, identify the pcrBias estimates that produce the minimum entropies in separating responders from non-responders that was calculated within the getPatientResponseEntropy Engine as the normalization bias factor for that feature. Alternatively, use the mean of the values for that non-Gx feature, over all patients in the Training Set, and identify that as the normalization bias factor for that feature.

2. For each non-Gx feature, calculate a modified standard deviation of values for that feature, centered around the bias factor used in step 1, over all patients in the Training Set, and identify that as the normalization scale factor for that feature. If the bias factor used is the mean then this modified standard deviation becomes the usual standard deviation.

3. For each non-Gx feature and patient, the normalized non-Gx data becomes:

$$\text{normalized non} - \text{Gx data} = \frac{(\text{original non}-\text{Gx data} - \text{The normalization bias factor for the feature})}{\text{The normalization scale factor for that feature}}$$

**The normalizePCRGxData Engine Algorithm**

The normalizePCRGxData Engine Algorithm takes the set of a patient's known PCR Gx data and normalizes it.
The algorithm is:

1. For each PCR Gx feature, identify the pcrBias estimates that produce the minimum entropies in separating responders from non-responders that was calculated within the getPatientResponseEntropy Engine as the normalization bias factor for that feature. Alternatively, use the mean of the values for that PCR Gx feature, over all patients in the Training Set, and identify that as the normalization bias factor for that feature.

2. For each PCR Gx feature, calculate a modified standard deviation of values for that feature, centered around the bias factor used in step 1, over all patients in the Training Set, and identify that as the normalization scale factor for that feature. If the bias factor used is the mean then this modified standard deviation becomes the usual standard deviation.

3. For each PCR Gx feature and patient, the normalized PCR Gx data becomes:

$$\text{normalized PCR Gx data} = \frac{(\text{original PCR Gx data} - \text{The normalization bias factor for the feature})}{\text{The normalization scale factor for that feature}}$$

**The getWeightedAndScaledTrainingMatrix Engine Algorithm**

In Training mode, for a given treatment response power as an input, the Training Set Patient Data Matrix is created by using the getWeightedAndScaledTrainingMatrix engine to perform a parameterized transformation of the prediction response data, then pull together all of the transformed data to form the Patient Data Matrix (X).
In non-Training mode (i.e. patient response prediction mode), treatment response power as an input, the Patient Data Matrix is created by using the getWeightedAndScaledTrainingMatrix engine to pull together all of the transformed (known Gx and non-Gx) data to form the Patient Data Matrix (X).
The algorithm is:

1. In the Training mode only, for the patient response rows from the normalized Patient Data Matrix, perform the following transformation:

$$\text{transformed patient response} = (\text{normalized patient response})^{\text{treatment response power}}$$

2. Pull together all of the transformed known Gx, known non-Gx, and, in Training mode only, the to be predicted patient response, data to form the Patient Data Matrix (X).

**The getLocalOptimClassifierParams Engine Algorithm**

As noted before, steps 3 and 4 of the workflow in Figure 5 perform parameterized re-scalings, biasing, and normalizations of the input data to transform it from a domain-specific "data space" into a less domain-specific "information space." We will refer to these data-transformation parameters as the Classifier Parameters. In one embodiment, typically 4 or less Classifier Parameters are required to characterize the Classifier System. A preferred embodiment uses just two such parameters. In an additional embodiment, two Classifier Parameters are required to characterize the Classifier System. These are the treatmentResponsePower and the effecPCREffic (effective PCR Efficiency). These parameters represent a low-order parameter characterization of the system. The global and constrained local classifier parameter optimization engines are used in the Algorithm Training System to determine good values for the data-transformation Classifier Parameters such as treatmentResponsePower and effecPCREffic.
The getLocalOptimClassifierParams Engine algorithm is:

1. Write the Classifier system in a parameterized form so that it takes Training Set data and the Classifier Parameters, such as treatmentResponsePower and effecPCREffic, as some of the inputs, and uses LOOCV (Leave One Out Cross Validation) to produce estimates of the fraction of correctly called responders and the fraction of correctly called non-responders.

2. For each set of Classifier Parameter values calculate the optimization objective as:

optimization objective

= sqrt(fraction of correctly called responders * the fraction of correctly called non-responders).

3. Starting from an initial set of values for the Classifier parameters, perform local parameter optimization to get the parameter set which maximizes the optimization objective. In a preferred embodiment this parameter optimization is a constrained parameter optimization.

**The getGlobalOptimClassifierParams Engine Algorithm**

The getGlobalOptimClassifierParams Engine algorithm is:

1. Write the Classifier system's getGlobalOptimClassifierParams Engine in a parameterized form so that it takes Training Set data and a starting set of Classifier Parameters, such as treatmentResponsePower and effecPCREffic, as some of the inputs, and uses LOOCV (Leave One Out Cross Validation) to produce an optimized set of Classifier parameters that produce a localized maximum in the optimization objective.

2. For a given starting set of Classifier Parameters produce an optimized set of Classifier parameters that produce a localized maximum in the optimization objective.

3. Use a Genetic Algorithm to produce an additional starting set of Classifier Parameters. Keep track of the set of Classifier Parameters that produce a global maximum in the optimization objective. Return to step 2 unless the maximum number of getGlobalOptimClassifierParams Engine algorithm iterations have been reached. Otherwise move on to step 4.

4. Return the set of Classifier Parameters that produce the global maximum in the optimization objective. Use these parameters to calculate and also return the inversion matrices that would be used to predict the patient response for patients outside of the Training Set.

In various embodiments, the methods of the present teachings may be implemented in a software program and applications written in conventional programming languages such as C, C++, etc.

While the present teachings are described in conjunction with various embodiments, it is not intended that the present teachings be limited to such embodiments. On the contrary, the present teachings encompass various alternatives, modifications, and equivalents, as will be appreciated by those of skill in the art.

The embodiments described herein, can be practiced with other computer system configurations including hand-held devices, microprocessor systems, microprocessor-based or programmable consumer electronics, minicomputers, mainframe computers and the like. The embodiments can also be practiced in distributing computing environments where tasks are performed by remote processing devices that are linked through a network.

It should also be understood that the embodiments described herein can employ various computer-implemented operations involving data stored in computer systems. These operations are those requiring physical manipulation of physical quantities. Usually, though not necessarily, these quantities take the form of electrical or magnetic signals capable of being stored, transferred, combined, compared, and otherwise manipulated. Further, the manipulations performed are often referred to in terms, such as producing, identifying, determining, or comparing.

Any of the operations that form part of the embodiments described herein are useful machine operations. The embodiments, described herein, also relate to a device or an apparatus for performing these operations. The systems and methods described herein can be specially constructed for the required purposes or it may be a general purpose computer selectively activated or configured by a computer program stored in the computer. In particular, various general purpose machines may be used with computer programs written in accordance with the teachings herein, or it may be more convenient to construct a more specialized apparatus to perform the required operations.

Certain embodiments can also be embodied as computer readable code on a computer readable medium. The computer readable medium is any data storage device that can store data, which can thereafter be read by a computer system. Examples of the computer readable medium include hard drives, network attached storage (NAS), read-only memory, random-access memory, CD-ROMs, CD-Rs, CD-RWs, magnetic tapes, and other optical, FLASH memory and non-optical data storage devices. The computer readable medium can also be distributed over a network coupled computer systems so that the computer readable code is stored and executed in a distributed fashion.

**Claims**

1. A system for predicting patient response to targeted therapies, comprising:

   a training data store configured to store a plurality of historical patient feature data records; and
   a gene expression analysis module networked with the training data store, the gene expression analysis module **characterized by**:

   a gene signature identification engine configured to:

   receive historical patient feature data records from the training data store, wherein the historical patient feature data records comprise information relating to gene expression and clinical response data, and identify therapeutically significant genomic signatures that are correlated to a particular medical condition and that are relevant to predicting a success parameter for a particular therapeutic regimen associated with the medical condition by applying a Principal Component Analysis (PCA) algorithm to the historical patient feature data records;

   and a predictive classifier engine configured to:

   receive a genomic signature and a proposed therapeutic regimen for a prospective patient,
   apply an Eigen-based classification algorithm to the genomic signature and the proposed therapeutic regimen to generate a predicted success parameter for the proposed therapeutic regimen based on the genomic signature of the prospective patient,
   wherein applying the Eigen based classification algorithm comprises associating the genomic signature of the prospective patient with the therapeutically significant genomic signatures identified by the gene signature identification engine,
   wherein the genomic signature and the therapeutically significant genomic signatures each represents a set of genes,
   wherein the predicted success parameter comprises a predicted cure rate, a predicted reduction of disease progression, or predicted patient lifespan increase for the prospective patient being treated with the proposed therapeutic regimen.

2. The system for predicting patient response to targeted therapies, as recited in claim 1, wherein the Principal Component Analysis (PCA) algorithm is a sparse Principal Component Analysis (PCA) algorithm.

3. The system for predicting patient response to targeted therapies, as recited in claim 1, further comprising:
   a genomic data capture system operable to capture a genomic signature of the prospective patient, wherein the genomic data capture system is networked with the gene expression analyzer.

4. The system for predicting patient response to targeted therapies, as recited in claim 1, wherein the genomic signature comprises one or more metagenes.

5. The system for predicting patient response to targeted therapies, as recited in claim 1, wherein the training data store and the gene expression analyzer reside on different computing devices.

6. The system for predicting patient response to targeted therapies, as recited in claim 1, wherein the historical patient feature data records comprises patient demographics, patient medical history, and patient lifestyle characteristics.

7. A computer implemented method for predicting patient response to targeted therapies, comprising:

   receiving historical patient feature data records from a training data store and **characterized by** identifying therapeutically significant genomic signatures that are correlated to a particular medical condition and that are relevant to predicting a success parameter for a particular therapeutic regimen associated with the medical condition by applying a Principal Component Analysis (PCA) algorithm to the historical patient feature data records, wherein the historical patient feature data records comprise information to gene expression and clinical response data;
   receiving a genomic signature and a proposed therapeutic regimen for a prospective patient; and
   applying an Eigen-based classification algorithm to the genomic signature and the proposed therapeutic regimen to generate a predicted success parameter for the proposed therapeutic regimen based on the genomic signature of the prospective patient,

wherein applying the Eigen based classification algorithm comprises associating the genomic signature of the prospective patient with the therapeutically significant genomic signatures,

wherein the genomic signature and the therapeutically significant genomic signatures each represents a set of genes,

wherein the predicted success parameter comprises a predicted cure rate, a predicted reduction of disease progression, or predicted patient lifespan increase for the prospective patient being treated with the proposed therapeutic regimen.

8. The computer-implemented method of claim 7, wherein the Principal Component Analysis (PCA) algorithm is a sparse Principal Component Analysis algorithm.

9. The computer-implemented method of claim 7, wherein the historical patient feature data records are modified to minimize the effects of domain specific biases and the Principal Component Analysis (PCA) algorithm is applied to the modified historical patient feature data records.

10. The computer-implemented method of claim 9, wherein the modification of the historical patient feature data records comprises scaling, normalizing, biasing, or weighting of the historical patient feature data records.

**Patentansprüche**

1. System zum Vorhersagen eines Ansprechens eines Patienten auf gezielte Therapien, das Folgendes umfasst:

einen Trainingsdatenspeicher, der konfiguriert ist, um mehrere historische Patientenmerkmalsdatensätze zu speichern; und

ein Genexpressionsanalysemodul, das mit dem Trainingsdatenspeicher vernetzt ist, wobei das Genexpressionsanalysemodul durch Folgendes gekennzeichnet ist:

eine Gensignaturidentifikations-Engine, die für Folgendes konfiguriert ist:

Empfangen von historischen Patientenmerkmalsdatensätzen aus dem Trainingsdatenspeicher, wobei die historischen Patientenmerkmalsdatensätze Informationen bezüglich der Genexpression und Daten des klinischen Ansprechens umfassen, und

Identifizieren therapeutisch signifikanter genomischer Signaturen, die mit einem speziellen medizinischen Zustand korreliert sind und die für das Vorhersagen eines Erfolgsparameters für einen speziellen Therapieplan relevant sind, der mit dem medizinischen Zustand verknüpft ist, durch Anwenden eines Hauptkomponentenanalyse(PCA - Principal Component Analysis)-Algorithmus auf die historischen Patientenmerkmalsdatensätze; und eine vorhersagende Klassifikator-Engine, die für Folgendes konfiguriert ist:

Empfangen einer genomischen Signatur und eines vorgeschlagenen Therapieplans für einen potenziellen Patienten,

Anwenden eines Eigen-basierten Klassifizierungsalgorithmus auf die genomische Signatur und den vorgeschlagenen Therapieplan, um einen vorhergesagten Erfolgsparameter für den vorgeschlagenen Therapieplan basierend auf der genomischen Signatur des potenziellen Patienten zu erzeugen,

wobei das Anwenden des Eigen-basierten Klassifizierungsalgorithmus das Verknüpfen der genomischen Signatur des potenziellen Patienten mit den therapeutisch signifikanten genomischen Signaturen umfasst, die durch die Gensignaturidentifikations-Engine identifiziert wurden,

wobei die genomische Signatur und die therapeutisch signifikanten genomischen Signaturen jeweils einen Satz von Genen darstellen,

wobei der vorhergesagte Erfolgsparameter eine vorhergesagte Heilungsrate, eine vorhergesagte Verringerung eines Krankheitsfortschritts oder eine vorhergesagte Erhöhung einer Patientenlebensdauer für den potenziellen Patienten umfasst, der mit dem vorgeschlagenen Therapieplan behandelt wird.

2. System zum Vorhersagen den Ansprechens des Patienten auf gezielte Therapien nach Anspruch 1, wobei der Hauptkomponentenanalyse(PCA)-Algorithmus ein dünnbesetzter Hauptkomponentenanalyse(PCA)-Algorithmus ist.

3. System zum Vorhersagen des Ansprechens des Patienten auf gezielte Therapien nach Anspruch 1, das ferner Folgendes umfasst:
ein genomisches Datenerfassungssystem, das betriebsfähig ist, um eine genomische Signatur des potenziellen Patienten zu erfassen, wobei das genomische Datenerfassungssystem mit dem Genexpressionsanalysator vernetzt ist.

**4.** System zum Vorhersagen des Ansprechens des Patienten auf gezielte Therapien nach Anspruch 1, wobei die genomische Signatur ein oder mehrere Metagene umfasst.

**5.** System zum Vorhersagen des Ansprechens des Patienten auf gezielte Therapien nach Anspruch 1, wobei sich der Trainingsdatenspeicher und der Genexpressionsanalysator auf verschiedenen Rechenvorrichtungen befinden.

**6.** System zum Vorhersagen des Ansprechens des Patienten auf gezielte Therapien nach Anspruch 1, wobei die historischen Patientenmerkmalsdatensätze Patientendemographie, Patientenanamnese und Eigenschaften des Lebensstils des Patienten umfassen.

**7.** Computerimplementiertes Verfahren zum Vorhersagen des Ansprechens des Patienten auf gezielte Therapien, das Folgendes umfasst:

Empfangen von historischen Patientenmerkmalsdatensätzen aus einem Trainingsdatenspeicher und das **gekennzeichnet ist durch**
Identifizieren von therapeutisch signifikanten genomischen Signaturen, die mit einem speziellen medizinischen Zustand korreliert sind und die für das Vorhersagen eines Erfolgsparameters für einen speziellen Therapieplan relevant sind, der mit dem medizinischen Zustand verknüpft ist, durch Anwenden eines Hauptkomponentenanalyse(PCA)-Algorithmus auf die historischen Patientenmerkmalsdatensätze, wobei die historischen Patientenmerkmalsdatensätze Informationen zu Genexpressions und Daten des klinischen Ansprechens umfassen;
Empfangen einer genomischen Signatur und eines vorgeschlagenen Therapieplans für einen potenziellen Patienten; und
Anwenden eines Eigen-basierten Klassifizierungsalgorithmus auf die genomische Signatur und den vorgeschlagenen Therapieplan, um einen vorhergesagten Erfolgsparameter für den vorgeschlagenen Therapieplan basierend auf der genomischen Signatur des potenziellen Patienten zu erzeugen,
wobei das Anwenden des Eigen-basierten Klassifizierungsalgorithmus das Verknüpfen der genomischen Signatur des potenziellen Patienten mit den therapeutisch signifikanten genomischen Signaturen umfasst,
wobei die genomische Signatur und die therapeutisch signifikanten genomischen Signaturen jeweils einen Satz von Genen darstellen,
wobei der vorhergesagte Erfolgsparameter eine vorhergesagte Heilungsrate, eine vorhergesagte Verringerung eines Krankheitsfortschritts oder eine vorhergesagte Erhöhung einer Patientenlebensdauer für den potenziellen Patienten umfasst, der mit dem vorgeschlagenen Therapieplan behandelt wird.

**8.** Computerimplementiertes Verfahren nach Anspruch 7, wobei der Hauptkomponentenanalyse(PCA)-Algorithmus ein dünnbesetzter Hauptkomponentenanalyse-Algorithmus ist.

**9.** Computerimplementiertes Verfahren nach Anspruch 7, wobei die historischen Patientenmerkmalsdatensätze modifiziert werden, um die Auswirkungen von domänenspezifischen Verzerrungen zu minimieren, und der Hauptkomponentenanalyse(PCA)-Algorithmus auf die modifizierten historischen Patientenmerkmalsdatensätze angewendet wird.

**10.** Computerimplementiertes Verfahren nach Anspruch 9, wobei die Modifikation der historischen Patientenmerkmalsdatensätze ein Skalieren, ein Normalisieren, ein Verzerren oder ein Gewichten der historischen Patientenmerkmalsdatensätze umfasst.

**Revendications**

**1.** Système destiné à prédire la réaction d'un patient à des traitements ciblés, comprenant :

une mémoire de données de formation configurée pour stocker une pluralité d'enregistrements de données historiques de caractéristiques de patient ; et
un module d'analyse de l'expression génique mis en réseau avec la mémoire de données de formation, le module d'analyse d'expression génique étant **caractérisé par** :

un moteur d'identification de signature génique configuré pour :

recevoir des enregistrements de données historiques de caractéristiques de patient à partir de la mémoire de données de formation, les enregistrements de données historiques de caractéristiques de patient comprenant des informations relatives à l'expression génique et des données de réaction clinique, et

identifier les signatures génomiques thérapeutiquement significatives qui sont corrélées à un trouble médical particulier et qui sont pertinentes dans le cadre de la prédiction d'un paramètre de succès d'un protocole de traitement particulier associé au trouble médical en appliquant un algorithme d'analyse en composantes principales (PCA) aux enregistrements de données historiques de caractéristiques du patient ;

et un moteur de classification prédictif configuré pour :

recevoir une signature génomique et une proposition de protocole de traitement pour un patient potentiel,

appliquer un algorithme de classification axé sur les vecteurs propres à la signature génomique et au protocole de traitement proposé pour générer un paramètre de succès prédit du protocole de traitement proposé sur la base de la signature génomique du patient potentiel,

l'application de l'algorithme de classification axé sur les vecteurs propres comprenant l'association de la signature génomique du patient potentiel aux signatures génomiques thérapeutiquement significatives identifiées par le moteur d'identification de signature génique,

la signature génomique et les signatures génomiques thérapeutiquement significatives représentant chacune un ensemble de gènes,

le paramètre de succès prédit comprenant un taux de guérison prédit, une réduction prédite de la progression de la maladie ou une augmentation de la durée de vie du patient prédite pour le patient potentiel traité à l'aide du protocole de traitement proposé.

**2.** Système destiné à prédire la réaction d'un patient à des traitements ciblés selon la revendication 1, l'algorithme d'analyse en composantes principales (PCA) étant un algorithme d'analyse en composantes principales (PCA) éparse.

**3.** Système destiné à prédire la réaction d'un patient à des traitements ciblés selon la revendication 1, comprenant en outre :
un système de capture de données génomiques pouvant être exploité pour capturer une signature génomique du patient potentiel, le système de capture de données génomiques étant mis en réseau avec l'analyseur d'expression génique.

**4.** Système destiné à prédire la réaction d'un patient à des traitements ciblés selon la revendication 1, la signature génomique comprenant un ou plusieurs métagènes.

**5.** Système destiné à prédire la réaction d'un patient à des traitements ciblés selon la revendication 1, la mémoire de données de formation et l'analyseur d'expression génique se trouvant sur différents dispositifs informatiques.

**6.** Système destiné à prédire la réaction d'un patient à des traitements ciblés selon la revendication 1, les enregistrements de données historiques de caractéristiques de patient comprenant des données démographiques du patient, des antécédents médicaux du patient et des caractéristiques du mode de vie du patient.

**7.** Procédé mis en oeuvre par ordinateur destiné à prédire la réaction d'un patient à des traitements ciblés, comprenant :

la réception d'enregistrements de données historiques de caractéristiques de patient à partir d'une mémoire de données de formation, et **caractérisé par**

l'identification de signatures génomiques thérapeutiquement significatives qui sont corrélées à un trouble médical particulier et qui sont pertinentes dans le cadre de la prédiction d'un paramètre de succès d'un protocole de traitement particulier associé au trouble médical en appliquant un algorithme d'analyse en composantes principales (PCA) aux enregistrements de données historiques de caractéristiques du patient, les enregistrements de données historiques de caractéristiques de patient comprenant des informations sur l'expression génique et des données de réaction clinique ;

la réception d'une signature génomique et d'un protocole de traitement proposé pour un patient potentiel ; et

l'application d'un algorithme de classification axé sur les vecteurs propres à la signature génomique et au protocole de traitement proposé pour générer un paramètre de succès prédit du protocole de traitement proposé sur la base de la signature génomique du patient potentiel,

l'application de l'algorithme de classification axé sur les vecteurs propres comprenant l'association de la signature génomique du patient potentiel aux signatures génomiques thérapeutiquement significatives,

la signature génomique et les signatures génomiques thérapeutiquement significatives représentant chacune un ensemble de gènes,

le paramètre de succès prédit comprenant un taux de guérison prédit, une réduction prédite de la progression de la maladie ou une augmentation de la durée de vie du patient prédite pour le patient potentiel traité à l'aide du protocole de traitement proposé.

8.  Procédé mis en oeuvre par ordinateur selon la revendication 7, l'algorithme d'analyse en composantes principales (PCA) étant un algorithme d'analyse en composantes principales éparse.

9.  Procédé mis en oeuvre par ordinateur selon la revendication 7, les enregistrements de données historiques de caractéristiques de patient étant modifiés pour minimiser les effets de biais propres au domaine et l'algorithme d'analyse en composantes principales (PCA) étant appliqué aux enregistrements de données historiques de caractéristiques de patient modifiés.

10.  Procédé mis en oeuvre par ordinateur selon la revendication 9, la modification des enregistrements de données historiques de caractéristiques de patient comprenant la mise à l'échelle, la normalisation, la soumission à un effet de biais ou la pondération des enregistrements de données historiques de caractéristiques de patient.

FIG. 1

FIG.2

**Analytics Computing Device/Server/Node**

300

Genomic Data Capture Device

304

302

**Gene Expression Analysis Module**

Gene Signature Identification Engine

310

Classifier Engine

312

302

Training Data Store

314

306

Display

316

**Client Device**

Data/Analysis API

318

Network

320

Public Genomic Medicine Content

322

Proprietary Genomic Medicine Content

324

EP 2 946 326 B1

29

**FIG.3**

310

404

402

| Classifier Parameters Global Optimization Engine | Controls → | Classifier Parameters Constrained Local Optimization Engine |

Iterative Loop

**FIG.4**

(1) Genomic (Cqs) and clinical data

(2) External "QC" data filtering

**Strong Data Dependency – Very Critical to Success**

502

(3) Data QC, filtering, and scaling
- Appropriate measure of affinity
- Log form (Cq) or Linear (Copy Number)
- Handle Noise

504

(4) Data entropy-based biasing, re-scaling, and normalization
- Handling Data Referencing (Housekeeping Genes)
- Etc.

506

(5) Gene Signature Selection
- Multivariate <- partially using Modified Sparse PCA Algorithm
- Leading to 3-6 metagenes (consisting of <= 41 genes total)

508

(6) Optionally Perform Probabilistic Clustering based on an underlying Bayesian framework
- Use prior information on training set patients' clinical response

510

(7) Perform Response Prediction using metagenes; Calc. quality score etc

$P_M, X'$
- Predict response to treatment
- Develop quality score
- Etc.

512

(8) LOOCV, LNOCV etc cross-validation
- Quality Scores, prediction metrics, prediction error variance,
- Parameter(s) Constrained Local Optimization using CODOSOL

514

$P_M, X'$

Prediction Matrix ($P_M$), Quality Scores, prediction metrics, etc

**FIG.5**

(1) Genomic (Cqs) and clinical data

(2) External "QC" data filtering

602

Strong Data Dependency – Very Critical to Success

604

$x_n$

(3) Data QC, filtering, and scaling
- Appropriate measure of affinity
- Log form (Cq) or Linear (Copy Number)
- Handle Noise

(4) Data entropy-based biasing, re-scaling, and normalization
- Handling Data Referencing (Housekeeping Genes)
- Etc.

606

608

$P_M, x'_n$

(7) Predict patient treatment response classification based on a multiple PC eigenvector inversion/prediction
- Calc quality score.

Patient Response Classification Prediction, Quality Scores,

FIG.6

| | | Sparse Eigenvectors (Down Columns) | | | | | | | | | | | | | | | | | | |
|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 | 18 | 19 | 20 |
| Pred. Resp. | 1 | | | | | | | | | | | | | | | | | | | | |
| Pred. Resp. | 2 | | | | | | | | | | | | | | | | | | | | |
| Known Gx or Feature | 1 | | | | | X | X | | | | | | | | | | | | | | X |
| Known Gx or Feature | 2 | | | | | | | X | | | | | | X | | X | | | | | |
| Known Gx or Feature | 3 | | | X | | | | | | | | | X | | X | | X | X | | X | |
| Known Gx or Feature | 4 | | | | | | | | | | | X | | | | | | | | | |
| Known Gx or Feature | 5 | | | | | | | | | | | | | | | | | | | | |
| Known Gx or Feature | 6 | X | | | | | | | | | | | | X | | | | | | | |
| Known Gx or Feature | 7 | | X | | | X | X | | | | X | ✓ | | | X | | X | | | X | |
| Known Gx or Feature | 8 | | | | | | | | | | | X | | | | X | | | | | |
| Known Gx or Feature | 9 | | | | | | | X | | | | | | | | | | | | | |
| Known Gx or Feature | 10 | | | | | | | | | | | X | | | | | | X | | | |
| Known Gx or Feature | 11 | X | | | | | X | | | | | | | | | | | | | X | X |
| Known Gx or Feature | 12 | | | | | | | | | | | | | | | | | | | | |
| Known Gx or Feature | 13 | | X | | X | | | | X | X | | X | | | | | | | | | |
| Known Gx or Feature | 14 | | | | | | | | | | | X | | | | | | | | | |
| Known Gx or Feature | 15 | | | | | | | | | | X | | | | | | | X | | | |
| Known Gx or Feature | 16 | | | | | | | | | | | X | | | | | | | | | |
| Known Gx or Feature | 17 | | X | | | | X | | X | | | | | | | | X | | | | |
| Known Gx or Feature | 18 | | X | X | | | | | | X | | | | | X | X | | | | | |
| Known Gx or Feature | 19 | | | | | | | | | | | | | | | | | | | | |
| Known Gx or Feature | 20 | X | | | | | | | | | | | | X | | | | | X | X | |
| Known Gx or Feature | 21 | | | | | | | X | X | | | | | | | X | | X | X | | |
| Known Gx or Feature | 22 | X | | | | | | | | | | | | | | | | | | | X |
| Known Gx or Feature | 23 | | | | | | | X | X | | | | X | | | | | | | | |
| Known Gx or Feature | 24 | | | | | | | | | | | | | | | | | | | | |
| Known Gx or Feature | 25 | | | | X | | | | X | X | | | | X | | | | X | | | |
| Known Gx or Feature | 26 | | | | | X | | | | | | | | | | | | | | | |
| Known Gx or Feature | 27 | X | X | | X | | | | | X | | | X | X | X | | | | | X | |
| Known Gx or Feature | 28 | | | | | | | | | X | | | | | | | | | | | |
| Known Gx or Feature | 29 | | | | | | | | | | | | | | | | | | | | |
| Known Gx or Feature | 30 | | | | | | | | | | X | | | | | | | | | | |
| Known Gx or Feature | 31 | | | | | X | | | | | | | X | X | | | | | X | | X |
| Known Gx or Feature | 32 | | | | | | | | | | | | | | | | | | | | |
| Known Gx or Feature | 33 | | | | | | X | | | | | | | | | | | | X | | |
| Known Gx or Feature | 34 | | | | | | | | | | | | | X | | | | X | | | X |
| Known Gx or Feature | 35 | | | X | | X | | X | | | | | | | | X | X | | | | |

**FIG.7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006084132 A
- US 87319010
- US 87313210
- US 2007066931
- US 2008003571, McKernan

**Non-patent literature cited in the description**

- **SAMBROOK et al.** Molecular Cloning: A Laboratory Manual. Cold Spring Harbor Laboratory Press, 2000
- **SIEGEL A. F. et al.** *Genomics,* 2000, vol. 68, 237-246
- **ROACH J. C. et al.** *Genomics,* 1995, vol. 26, 345-353
- **MILLIKIN S. C. et al.** *Genome Res,* 2003, vol. 13, 81-90
- **KENT, W.J. et al.** *Genome Res,* 2001, vol. 11, 1541-8